# EUROPEAN PATENT APPLICATION

(11) **EP 1 156 062 A1**
(43) Date of publication of application: **21.11.2001**
(21) Application number: 00110063.5
(22) Date of filing: 12.05.2000
(51) Int. Cl.: C07K 16/28, C12N 15/13, C12N 1/21, C12N 5/10, C12N 15/70, C12N 15/85, A61K 39/395, A61P 37/00, G01N 33/68, G01N 33/53, G01N 33/577, C12Q 1/68

(54) **Immunomodulatory human MHC class II antigen-binding peptides/proteins**

(71) Applicant: GPC Biotech AG, 82152 Martinsried (DE); MorphoSys AG, 82152 Martinsried (DE)
(72) Inventor: Zoltan, Nagy, Cambridge, MA 02139 (US); Thomassen-Wolf, Elisabeth, 81369 München (DE); Tesar, Michael, 82362 Weilheim (DE)
(74) Representative: Horner, Martin Grenville

(57) **Abstract**

The present invention relates to human peptides/proteins comprising at least one antibody-based antigen-binding domain of human composition with a binding specificity for a human MHC class II antigen, wherein binding of said peptide/protein to said antigen expressed on the surface of a cell causes or leads to modulation of the immune system. The invention further relates to nucleic acids encoding said peptides/proteins, methods for production, immunosuppression, pharmaceutical and diagnostic compositions or kits comprising the peptides/proteins and uses of the peptides/proteins.

### Annex to the application documents-subsequently filed sequences listing

SEQUENCE LISTING

<110> GPC Biotech AG
      MorphoSys AG

<120>  Immunomodulatory human MHC class II antigen-binding
       peptides/proteins

<130>  modulating antibody

<140>  00110063.5
<141>  2000-05-12

<160>  65

<170>  PatentIn version 3.0

<210>  1
<211>  10
<212>  PRT
<213>  artificial sequence

<220>
<221>  VHconCDR3
<222>  (1)..(10)
<223>  "X" represents any amino acid residue


<400>  1

Xaa Xaa Xaa Xaa Arg Gly Xaa Phe Asp Xaa
1               5                   10

<210>  2
<211>  8
<212>  PRT
<213>  artificial sequence

<220>
<221>  VLconCDR3
<222>  (1)..(8)
<223>  "X" represents any amino acid residue


<400>  2

Gln Ser Tyr Asp Xaa Xaa Xaa Xaa
1               5

<210>  3
<211>  8
<212>  PRT
<213>  artificial sequence

<220>
<221>  Streptaq
<222>  (1)..(8)

<400>  3

Trp Ser His Pro Gln Phe Glu Lys
1               5

<210>  4
<211>  17
<212>  DNA
<213>  artificial sequence

<220>
<221>  PrimerCRT5
<222>  (1)..(17)

<400>  4
gtggtggttc cgatatc                                                    17


<210>  5
<211>  44
<212>  DNA
<213>  artificial sequence

<220>
<221>  PrimerCRT6
<222>  (1)..(44)

<400>  5
agcgtcacac tcggtgcggc tttcggctgg ccaagaacgg gtta                      44


<210>  6
<211>  10
<212>  PRT
<213>  artificial sequence

<220>
<221>  MS-GPC1-VH-CDR3
<222>  (1)..(10)

<400>  6

Gln Tyr Gly His Arg Gly Gly Phe Asp His
1               5                   10

<210>  7
<211>  8
<212>  PRT
<213>  artificial sequence

<220>
<221>  MS-GPC1-VL-CDR3
<222>  (1)..(8)

<400>  7

Gln Ser Tyr Asp Phe Asn Glu Ser
1               5

<210>  8
<211>  19
<212>  PRT
<213>  artificial sequence

<220>
<221>  MS-GPC2-VH-CDR3
<222>  (1)..(19)

<400>  8

Ser His Asn Lys Lys Trp Arg Phe Tyr Asn Leu Tyr Ser Leu Tyr Asp
1               5                   10                  15

Phe Asp Phe


<210>  9
<211>  8
<212>  PRT
<213>  artificial sequence

<220>
<221>  MS-GPC2-VL-CDR3
<222>  (1)..(8)

<400>  9

Gln Gln Glu Ser Gly Phe Pro Tyr
1               5

<210>  10
<211>  7
<212>  PRT
<213>  artificial sequence

<220>
<221>  MS-GPC3-VH-CDR3
<222>  (1)..(7)

<400>  10

Leu Ser Thr Arg Met Asp Pro
1               5

<210>  11
<211>  8
<212>  PRT
<213>  artificial sequence

<220>
<221>  MS-GPC3-VL-CDR3
<222>  (1)..(8)

<400>  11

Gln Gln Asp Asp Asn Phe Pro Ile
1               5

<210>  12
<211>  16
<212>  PRT
<213>  artificial sequence

<220>
<221>  MS-GPC4-VH-CDR3
<222>  (1)..(16)

<400>  12

Tyr Tyr Val Tyr Ser Val Gly Tyr Gly Val Thr His Tyr Asp Asp Val
1               5                   10                  15

<210>  13
<211>  8
<212>  PRT
<213>  artificial sequence

<220>
<221>  MS-GPC4-VL-CDR3
<222>  (1)..(8)

<400>  13

Gln Gln Asp Tyr Ser Tyr Pro Ser
1               5

<210>  14
<211>  6
<212>  PRT
<213>  artificial sequence

<220>
<221>  MS-GPC5VH-CDR3
<222>  (1)..(6)

<400>  14

His Ser Phe Phe Asp Tyr
1               5

<210>  15
<211>  9
<212>  PRT
<213>  artificial sequence

<220>
<221>  MS-GPC5-VL-CDR3
<222>  (1)..(9)

<400>  15

Gln Ser Tyr Asp Asn Val Asp Ile Ser
1               5

<210>  16
<211>  9
<212>  PRT
<213>  artificial sequence

<220>
<221>  MS-GPC6-VH-CDR3
<222>  (1)..(9)

<400>  16

Gly Tyr Gly Arg Tyr Ser Pro Asp Leu
1               5

<210>  17
<211>  8
<212>  PRT
<213>  artificial sequence

<220>

<221>  MS-GPC6-VL-CDR3
<222>  (1)..(8)

<400>  17

Gln Gln Tyr Ser Asn Leu Pro Phe
1               5

<210>  18
<211>  12
<212>  PRT
<213>  artificial sequence

<220>
<221>  MS-GPC7-VH-CDR3
<222>  (1)..(12)

<400>  18

Ser Gln Asn Gly Phe Tyr Gly Gly Asn Leu Asp Ile
1               5                   10

<210>  19
<211>  8
<212>  PRT
<213>  artificial sequence

<220>
<221>  MS-GPC7-VL-CDR3
<222>  (1)..(8)

<400>  19

Gln Ser Arg Asp Pro Ser Asn Val
1               5

<210>  20
<211>  10
<212>  PRT
<213>  artificial sequence

<220>
<221>  MS-GPC8-VH-CDR3
<222>  (1)..(10)

<400>  20

Ser Pro Arg Tyr Arg Gly Ala Phe Asp Tyr
1               5                   10

<210>  21
<211>  8
<212>  PRT
<213>  artificial sequence

<220>
<221>  MS-GPC8-VL-CDR3
<222>  (1)..(8)

<400>  21

Gln Ser Tyr Asp Met Pro Gln Ala
1               5

<210>  22
<211>  10
<212>  PRT
<213>  artificial sequence

<220>
<221>  MS-GPC10-VH-CDR3
<222>  (1)..(10)

<400>  22

Gln Leu His Tyr Arg Gly Gly Phe Asp Leu
1               5                   10

<210>  23
<211>  8
<212>  PRT
<213>  artificial sequence

<220>
<221>  MS-GPC10-VL-CDR3
<222>  (1)..(8)

<400>  23

Gln Ser Tyr Asp Leu Thr Met Gly
1               5

<210>  24
<211>  10
<212>  PRT
<213>  artificial sequence

<220>
<221>  MS-GPC11-VH-CDR3
<222>  (1)..(10)

<400>  24

Ser Gln Gly Tyr Arg Gly Gly Leu Asp Val
1               5                   10

<210>  25
<211>  8
<212>  PRT
<213>  artificial sequence

<220>
<221>  MS-GPC11-VL-CDR3
<222>  (1)..(8)

<400>  25

Gln Ser Tyr Asp Tyr Gly Ile Tyr
1               5

<210>  26
<211>  12
<212>  PRT
<213>  artificial sequence

<220>

<221>  MS-GPC14-VH-CDR3
<222>  (1)..(12)

<400>  26

Ser Ser Met Pro Met Tyr Gly Glu Gly Phe Asp Leu
1               5                   10

<210>  27
<211>  10
<212>  PRT
<213>  artificial sequence

<220>
<221>  MS-GPC14-VL-CDR3
<222>  (1)..(10)

<400>  27

Gln Ser Tyr Asp Phe Gly Val Ser His Ser
1               5                   10

<210>  28
<211>  11
<212>  PRT
<213>  artificial sequence

<220>
<221>  MS-GPC15-VH-CDR3
<222>  (1)..(11)

<400>  28

Phe Tyr Tyr Ser His Val Leu Ala Met Asp Asn
1               5                   10

<210>  29
<211>  10
<212>  PRT
<213>  artificial sequence

<220>
<221>  MS-GPC15-VL-CDR3
<222>  (1)..(10)

<400>  29

Gln Ser Arg Asp Ile His Ile His Asn Glu
1               5                   10

<210>  30
<211>  8
<212>  PRT
<213>  artificial sequence

<220>
<221>  MS-GPC16-VH-CDR3
<222>  (1)..(8)

<400>  30

Thr Gln Leu Tyr Tyr Phe Asp Tyr
1               5

<210> 31
<211> 8
<212> PRT
<213> artificial sequence

<220>
<221> MS-GPC16-VL-CDR3
<222> (1)..(8)

<400> 31

Gln Gln Tyr Asn Ser Tyr Pro Arg
1               5

<210> 32
<211> 8
<212> PRT
<213> artificial sequence

<220>
<221> MS-GPC8-6-VL-CDR3
<222> (1)..(8)

<400> 32

Gln Ser Tyr Asp Tyr Asp His Tyr
1               5

<210> 33
<211> 8
<212> PRT
<213> artificial sequence

<220>
<221> MS-GPC8-17-VL-CDR3
<222> (1)..(8)

<400> 33

Gln Ser Tyr Asp Phe Ser Val Tyr
1               5

<210> 34
<211> 3548
<212> DNA
<213> artificial sequence

<220>
<221> pMORPH13_scFv
<222> (1)..(3548)

<400> 34
agagcatgcg taggagaaaa taaaatgaaa caaagcacta ttgcactggc actcttaccg     60

ttgctcttca cccctgttac caaagccgac tacaaagatg aagtgcaatt ggttcagtct    120

ggcgcggaag tgaaaaaacc gggcagcagc gtgaaagtga gctgcaaagc ctccggaggc    180

acttttagca gctatgcgat tagctgggtg cgccaagccc ctgggcaggg tctcgagtgg    240

atgggcggca ttattccgat ttttggcacg gcgaactacg cgcagaagtt tcagggccgg    300

gtgaccatta ccgcggatga aagcaccagc accgcgtata tggaactgag cagcctgcgt 360
agcgaagata cggccgtgta ttattgcgcg cgttattatg atcgtatgta taatatggat 420
tattggggcc aaggcaccct ggtgacggtt agctcagcgg gtggcggttc tggcggcggt 480
gggagcggtg gcggtggttc tggcggtggt ggttccgata tcgaactgac ccagccgcct 540
tcagtgagcg ttgcaccagg tcagaccgcg cgtatctcgt gtagcggcga tgcgctgggc 600
gataaatacg cgagctggta ccagcagaaa cccgggcagg cgccagttct ggtgatttat 660
gatgattctg accgtccctc aggcatcccg gaacgcttta gcggatccaa cagcggcaac 720
accgcgaccc tgaccattag cggcactcag gcggaagacg aagcggatta ttattgccag 780
agctatgacg ctcatatgcg tcctgtgttt ggcggcggca cgaagttaac cgttcttggc 840
caggaattcg agcagaagct gatctctgag gaggatctga actagggtgg tggctctggt 900
tccggtgatt ttgattatga aaagatggca aacgctaata agggggctat gaccgaaaat 960
gccgatgaaa acgcgctaca gtctgacgct aaaggcaaac ttgattctgt cgctactgat 1020
tacggtgctg ctatcgatgg tttcattggt gacgtttccg gccttgctaa tggtaatggt 1080
gctactggtg attttgctgg ctctaattcc caaatggctc aagtcggtga cggtgataat 1140
tcacctttaa tgaataattt ccgtcaatat ttaccttccc tccctcaatc ggttgaatgt 1200
cgcccttttg tctttggcgc tggtaaacca tatgaatttt ctattgattg tgacaaaata 1260
aacttattcc gtggtgtctt tgcgtttctt ttatatgttg ccacctttat gtatgtattt 1320
tctacgtttg ctaacatact gcgtaataag gagtcttgat aagcttgacc tgtgaagtga 1380
aaaatggcgc agattgtgcg acattttttt tgtctgccgt ttaatgaaat tgtaaacgtt 1440
aatattttgt taaaattcgc gttaaatttt tgttaaatca gctcattttt taaccaatag 1500
gccgaaatcg gcaaaatccc ttataaatca aaagaataga ccgagatagg gttgagtgtt 1560
gttccagttt ggaacaagag tccactatta aagaacgtgg actccaacgt caaagggcga 1620
aaaaccgtct atcagggcga tggcccacta cgagaaccat caccctaatc aagttttttg 1680
gggtcgaggt gccgtaaagc actaaatcgg aaccctaaag ggagcccccg atttagagct 1740
tgacggggaa agccggcgaa cgtggcgaga aaggaaggga agaaagcgaa aggagcgggc 1800
gctagggcgc tggcaagtgt agcggtcacg ctgcgcgtaa ccaccacacc cgccgcgctt 1860
aatgcgccgc tacagggcgc gtgctagcca tgtgagcaaa aggccagcaa aaggccagga 1920
accgtaaaaa ggccgcgttg ctggcgtttt tccataggct ccgcccccct gacgagcatc 1980
acaaaaatcg acgctcaagt cagaggtggc gaaacccgac aggactataa agataccagg 2040
cgtttccccc tggaagctcc ctcgtgcgct ctcctgttcc gaccctgccg cttaccggat 2100
acctgtccgc ctttctccct tcgggaagcg tggcgctttc tcatagctca cgctgtaggt 2160
atctcagttc ggtgtaggtc gttcgctcca agctgggctg tgtgcacgaa ccccccgttc 2220

agtccgaccg ctgcgcctta tccggtaact atcgtcttga gtccaacccg gtaagacacg 2280

acttatcgcc actggcagca gccactggta acaggattag cagagcgagg tatgtaggcg 2340

gtgctacaga gttcttgaag tggtggccta actacggcta cactagaaga acagtatttg 2400

gtatctgcgc tctgctgtag ccagttacct tcggaaaaag agttggtagc tcttgatccg 2460

gcaaacaaac caccgctggt agcggtggtt tttttgtttg caagcagcag attacgcgca 2520

gaaaaaaagg atctcaagaa gatcctttga tcttttctac ggggtctgac gctcagtgga 2580

acgaaaactc acgttaaggg attttggtca gatctagcac caggcgttta agggcaccaa 2640

taactgcctt aaaaaaatta cgccccgccc tgccactcat cgcagtactg ttgtaattca 2700

ttaagcattc tgccgacatg gaagccatca caaacggcat gatgaacctg aatcgccagc 2760

ggcatcagca ccttgtcgcc ttgcgtataa tatttgccca tagtgaaaac gggggcgaag 2820

aagttgtcca tattggctac gtttaaatca aaactggtga aactcaccca gggattggct 2880

gagacgaaaa acatattctc aataaaccct ttagggaaat aggccaggtt ttcaccgtaa 2940

cacgccacat cttgcgaata tatgtgtaga aactgccgga aatcgtcgtg gtattcactc 3000

cagagcgatg aaaacgtttc agtttgctca tggaaaacgg tgtaacaagg gtgaacacta 3060

tcccatatca ccagctcacc gtctttcatt gccatacgga actccgggtg agcattcatc 3120

aggcgggcaa gaatgtgaat aaaggccgga taaaacttgt gcttattttt ctttacggtc 3180

tttaaaaagg ccgtaatatc cagctgaacg gtctggttat aggtacattg agcaactgac 3240

tgaaatgcct caaaatgttc tttacgatgc cattgggata tatcaacggt ggtatatcca 3300

gtgatttttt tctccatttt agcttcctta gctcctgaaa atctcgataa ctcaaaaaat 3360

acgcccggta gtgatcttat ttcattatgg tgaaagttgg aacctcaccc gacgtctaat 3420

gtgagttagc tcactcatta ggcaccccag gctttacact ttatgcttcc ggctcgtatg 3480

ttgtgtggaa ttgtgagcgg ataacaattt cacacaggaa acagctatga ccatgattac 3540

gaatttct 3548

<210> 35
<211> 4410
<212> DNA
<213> artificial sequence

<220>
<221> pMx7_FS_5D2
<222> (1)..(4410)

<400> 35
tctagagcat gcgtaggaga aaataaaatg aaacaaagca ctattgcact ggcactctta 60

ccgttgctct tcacccctgt taccaaagcc gactacaaag atgaagtgca attggtggaa 120

agcggcggcg gcctggtgca accgggcggc agcctgcgtc tgagctgcgc ggcctccgga 180

tttaccttta gcagctatgc gatgagctgg gtgcgccaag cccctgggaa gggtctcgag 240

tgggtgagcg cgattagcgg tagcggcggc agcacctatt atgcggatag cgtgaaaggc 300

cgttttacca tttcacgtga taattcgaaa aacaccctgt atctgcaaat gaacagcctg 360

cgtgcggaag atacggccgt gtattattgc gcgcgtgtta agaagcattt ttctcgtaag 420

aattggtttg attattgggg ccaaggcacc ctggtgacgg ttagctcagc gggtggcggt 480

tctggcggcg gtgggagcgg tggcggtggt tctggcggtg gtggttccga tatcgtgatg 540

acccagagcc cactgagcct gccagtgact ccgggcgagc ctgcgagcat tagctgcaga 600

agcagccaaa gcctgctgca tagcaacggc tataactatc tggattggta ccttcaaaaa 660

ccaggtcaaa gcccgcagct attaatttat ctgggcagca accgtgccag tggggtcccg 720

gatcgtttta gcggctctgg atccggcacc gattttaccc tgaaaattag ccgtgtggaa 780

gctgaagacg tgggcgtgta ttattgccag cagcattata ccaccccgcc gacctttggc 840

cagggtacga aagttgaaat taaacgtacg gaattcgact ataaagatga cgatgacaaa 900

ggcgcgccgt ggagccaccc gcagtttgaa aaatgataag cttgacctgt gaagtgaaaa 960

atggcgcaga ttgtgcgaca ttttttttgt ctgccgttta attaaagggg ggggggggcc 1020

ggcctggggg ggggtgtaca tgaaattgta aacgttaata ttttgttaaa attcgcgtta 1080

aatttttgtt aaatcagctc attttttaac caataggccg aaatcggcaa aatcccttat 1140

aaatcaaaag aatagaccga gatagggttg agtgttgttc cagtttggaa caagagtcca 1200

ctattaaaga acgtggactc caacgtcaaa gggcgaaaaa ccgtctatca gggcgatggc 1260

ccactacgag aaccatcacc ctaatcaagt tttttggggt cgaggtgccg taaagcacta 1320

aatcggaacc ctaaagggag cccccgattt agagcttgac ggggaaagcc ggcgaacgtg 1380

gcgagaaagg aagggaagaa agcgaaagga gcgggcgcta gggcgctggc aagtgtagcg 1440

gtcacgctgc gcgtaaccac cacacccgcc gcgcttaatg cgccgctaca gggcgcgtgc 1500

tagactagtg tttaaaccgg accggggggg ggcttaagtg ggctgcaaaa caaaacggcc 1560

tcctgtcagg aagccgcttt tatcgggtag cctcactgcc cgctttccag tcgggaaacc 1620

tgtcgtgcca gctgcatcag tgaatcggcc aacgcgcggg gagaggcggt ttgcgtattg 1680

ggagccaggg tggtttttct tttcaccagt gagacgggca acagctgatt gcccttcacc 1740

gcctggccct gagagagttg cagcaagcgg tccacgctgg tttgccccag caggcgaaaa 1800

tcctgtttga tggtggtcag cggcgggata taacatgagc tgtcctcggt atcgtcgtat 1860

cccactaccg agatgtccgc accaacgcgc agcccggact cggtaatggc acgcattgcg 1920

cccagcgcca tctgatcgtt ggcaaccagc atcgcagtgg gaacgatgcc ctcattcagc 1980

atttgcatgg tttgttgaaa accggacatg gcactccagt cgccttcccg ttccgctatc 2040

ggctgaattt gattgcgagt gagatattta tgccagccag ccagacgcag acgcgccgag 2100

acagaactta atgggccagc taacagcgcg atttgctggt ggcccaatgc gaccagatgc 2160

tccacgccca gtcgcgtacc gtcctcatgg gagaaaataa tactgttgat gggtgtctgg 2220

tcagagacat caagaaataa cgccggaaca ttagtgcagg cagcttccac agcaatagca 2280

tcctggtcat ccagcggata gttaataatc agcccactga cacgttgcgc gagaagattg 2340

tgcaccgccg ctttacaggc ttcgacgccg cttcgttcta ccatcgacac gaccacgctg 2400

gcacccagtt gatcggcgcg agatttaatc gccgcgacaa tttgcgacgg cgcgtgcagg 2460

gccagactgg aggtggcaac gccaatcagc aacgactgtt tgcccgccag ttgttgtgcc 2520

acgcggttag gaatgtaatt cagctccgcc atcgccgctt ccactttttc ccgcgttttc 2580

gcagaaacgt ggctggcctg gttcaccacg cgggaaacgg tctgataaga gacaccggca 2640

tactctgcga catcgtataa cgttactggt ttcacattca ccaccctgaa ttgactctct 2700

tccgggcgct atcatgccat accgcgaaag gttttgcgcc attcgatgct agccatgtga 2760

gcaaaaggcc agcaaaaggc caggaaccgt aaaaaggccg cgttgctggc gtttttccat 2820

aggctccgcc cccctgacga gcatcacaaa aatcgacgct caagtcagag gtggcgaaac 2880

ccgacaggac tataaagata ccaggcgttt ccccctggaa gctccctcgt gcgctctcct 2940

gttccgaccc tgccgcttac cggatacctg tccgcctttc tcccttcggg aagcgtggcg 3000

ctttctcata gctcacgctg taggtatctc agttcggtgt aggtcgttcg ctccaagctg 3060

ggctgtgtgc acgaaccccc cgttcagccc gaccgctgcg ccttatccgg taactatcgt 3120

cttgagtcca acccggtaag acacgactta tcgccactgg cagcagccac tggtaacagg 3180

attagcagag cgaggtatgt aggcggtgct acagagttct tgaagtggtg gcctaactac 3240

ggctacacta gaagaacagt atttggtatc tgcgctctgc tgtagccagt taccttcgga 3300

aaaagagttg gtagctcttg atccggcaaa caaaccaccg ctggtagcgg tggttttttt 3360

gtttgcaagc agcagattac gcgcagaaaa aaaggatctc aagaagatcc tttgatcttt 3420

tctacggggt ctgacgctca gtggaacgaa aactcacgtt aagggatttt ggtcagatct 3480

agcaccaggc gtttaagggc accaataact gccttaaaaa aattacgccc cgccctgcca 3540

ctcatcgcag tactgttgta attcattaag cattctgccg acatggaagc catcacaaac 3600

ggcatgatga acctgaatcg ccagcggcat cagcaccttg tcgccttgcg tataatattt 3660

gcccatagtg aaaacggggg cgaagaagtt gtccatattg gctacgttta aatcaaaact 3720

ggtgaaactc acccagggat tggctgagac gaaaaacata ttctcaataa accctttagg 3780

gaaataggcc aggttttcac cgtaacacgc cacatcttgc gaatatatgt gtagaaactg 3840

ccggaaatcg tcgtggtatt cactccagag cgatgaaaac gtttcagttt gctcatggaa 3900

aacggtgtaa caagggtgaa cactatccca tatcaccagc tcaccgtctt tcattgccat 3960

acggaactcc gggtgagcat tcatcaggcg ggcaagaatg tgaataaagg ccggataaaa 4020

cttgtgctta tttttcttta cggtctttaa aaaggccgta atatccagct gaacggtctg 4080

gttataggta cattgagcaa ctgactgaaa tgcctcaaaa tgttctttac gatgccattg 4140

ggatatatca acggtggtat atccagtgat ttttttctcc attttagctt ccttagctcc 4200

tgaaaatctc gataactcaa aaaatacgcc cggtagtgat cttatttcat tatggtgaaa 4260

gttggaacct cacccgacgt ctaatgtgag ttagctcact cattaggcac cccaggcttt 4320

acactttatg cttccggctc gtatgttgtg tggaattgtg agcggataac aatttcacac 4380

aggaaacagc tatgaccatg attacgaatt 4410

<210> 36
<211> 5020
<212> DNA
<213> artificial sequence

<220>
<221> pMx9_Fab_GPC8
<222> (1)..(5020)

<400> 36
atcgtgctga cccagccgcc ttcagtgagt ggcgcaccag gtcagcgtgt gaccatctcg 60

tgtagcggca gcagcagcaa cattggcagc aactatgtga gctggtacca gcagttgccc 120

gggacggcgc cgaaactgct gatttatgat aacaaccagc gtccctcagg cgtgccggat 180

cgttttagcg gatccaaaag cggcaccagc gcgagccttg cgattacggg cctgcaaagc 240

gaagacgaag cggattatta ttgccagagc tatgacatgc ctcaggctgt gtttggcggc 300

ggcacgaagt ttaaccgttc ttggccagcc gaaagccgca ccgagtgtga cgctgtttcc 360

gccgagcagc gaagaattgc aggcgaacaa agcgaccctg gtgtgcctga ttagcgactt 420

ttatccggga gccgtgacag tggcctggaa ggcagatagc agccccgtca aggcgggagt 480

ggagaccacc acaccctcca aacaaagcaa caacaagtac gcggccagca gctatctgag 540

cctgacgcct gagcagtgga agtcccacag aagctacagc tgccaggtca cgcatgaggg 600

gagcaccgtg gaaaaaaccg ttgcgccgac tgaggcctga taagcatgcg taggagaaaa 660

taaaatgaaa caaagcacta ttgcactggc actcttaccg ttgctcttca cccctgttac 720

caaagcccag gtgcaattga aagaaagcgg cccggccctg gtgaaaccga cccaaaccct 780

gaccctgacc tgtacctttt ccggatttag cctgtccacg tctggcgttg gcgtgggctg 840

gattcgccag ccgcctggga aagccctcga gtggctggct ctgattgatt gggatgatga 900

taagtattat agcaccagcc tgaaaacgcg tctgaccatt agcaaagata cttcgaaaaa 960

tcaggtggtg ctgactatga ccaacatgga cccggtggat acggccacct attattgcgc 1020

gcgttctcct cgttatcgtg gtgcttttga ttattggggc caaggcaccc tggtgacggt 1080

tagctcagcg tcgaccaaag gtccaagcgt gtttccgctg gctccgagca gcaaaagcac 1140

cagcggcggc acggctgccc tgggctgcct ggttaaagat tatttcccgg aaccagtcac 1200

cgtgagctgg aacagcgggg cgctgaccag cggcgtgcat acctttccgg cggtgctgca 1260

aagcagcggc ctgtatagcc tgagcagcgt tgtgaccgtg ccgagcagca gcttaggcac 1320

tcagacctat atttgcaacg tgaaccataa accgagcaac accaaagtgg ataaaaaagt 1380

ggaaccgaaa agcgaattcg actataaaga tgacgatgac aaaggcgcgc cgtggagcca 1440

cccgcagttt gaaaaatgat aagcttgacc tgtgaagtga aaaatggcgc agattgtgcg 1500

acattttttt tgtctgccgt ttaattaaag gggggggggg gccggcctgg gggggggtgt 1560

acatgaaatt gtaaacgtta atattttgtt aaaattcgcg ttaaattttt gttaaatcag 1620

ctcatttttt aaccaatagg ccgaaatcgg caaaatccct tataaatcaa aagaatagac 1680

cgagataggg ttgagtgttg ttccagtttg gaacaagagt ccactattaa agaacgtgga 1740

ctccaacgtc aaagggcgaa aaaccgtcta tcagggcgat ggcccactac gagaaccatc 1800

accctaatca agttttttgg ggtcgaggtg ccgtaaagca ctaaatcgga accctaaagg 1860

gagcccccga tttagagctt gacggggaaa gccggcgaac gtggcgagaa aggaagggaa 1920

gaaagcgaaa ggagcgggcg ctagggcgct ggcaagtgta gcggtcacgc tgcgcgtaac 1980

caccacaccc gccgcgctta atgcgccgct acagggcgcg tgctagacta gtgtttaaac 2040

cggaccgggg gggggcttaa gtgggctgca aaacaaaacg gcctcctgtc aggaagccgc 2100

ttttatcggg tagcctcact gcccgctttc cagtcgggaa acctgtcgtg ccagctgcat 2160

cagtgaatcg gccaacgcgc ggggagaggc ggtttgcgta ttgggagcca gggtggtttt 2220

tcttttcacc agtgagacgg gcaacagctg attgcccttc accgcctggc cctgagagag 2280

ttgcagcaag cggtccacgc tggtttgccc cagcaggcga aaatcctgtt tgatggtggt 2340

cagcggcggg atataacatg agctgtcctc ggtatcgtcg tatcccacta ccgagatgtc 2400

cgcaccaacg cgcagcccgg actcggtaat ggcacgcatt gcgcccagcg ccatctgatc 2460

gttggcaacc agcatcgcag tgggaacgat gccctcattc agcatttgca tggtttgttg 2520

aaaaccggac atggcactcc agtcgccttc ccgttccgct atcggctgaa tttgattgcg 2580

agtgagatat ttatgccagc cagccagacg cagacgcgcc gagacagaac ttaatgggcc 2640

agctaacagc gcgatttgct ggtggcccaa tgcgaccaga tgctccacgc ccagtcgcgt 2700

accgtcctca tgggagaaaa taatactgtt gatgggtgtc tggtcagaga catcaagaaa 2760

taacgccgga acattagtgc aggcagcttc cacagcaata gcatcctggt catccagcgg 2820

atagttaata atcagcccac tgacacgttg cgcgagaaga ttgtgcaccg ccgctttaca 2880

ggcttcgacg ccgcttcgtt ctaccatcga cacgaccacg ctggcaccca gttgatcggc 2940

gcgagattta atcgccgcga caatttgcga cggcgcgtgc agggccagac tggaggtggc 3000

aacgccaatc agcaacgact gtttgcccgc cagttgttgt gccacgcggt taggaatgta 3060

attcagctcc gccatcgccg cttccacttt ttcccgcgtt ttcgcagaaa cgtggctggc 3120

ctggttcacc acgcgggaaa cggtctgata agagacaccg gcatactctg cgacatcgta 3180

taacgttact ggtttcacat tcaccaccct gaattgactc tcttccgggc gctatcatgc 3240

cataccgcga aaggttttgc gccattcgat gctagccatg tgagcaaaag gccagcaaaa 3300

ggccaggaac cgtaaaaagg ccgcgttgct ggcgtttttc cataggctcc gcccccctga 3360

cgagcatcac aaaaatcgac gctcaagtca gaggtggcga aacccgacag gactataaag 3420

ataccaggcg tttccccctg gaagctccct cgtgcgctct cctgttccga ccctgccgct 3480

taccggatac ctgtccgcct ttctcccttc gggaagcgtg gcgctttctc atagctcacg 3540

ctgtaggtat ctcagttcgg tgtaggtcgt tcgctccaag ctgggctgtg tgcacgaacc 3600

ccccgttcag cccgaccgct gcgccttatc cggtaactat cgtcttgagt ccaacccggt 3660

aagacacgac ttatcgccac tggcagcagc cactggtaac aggattagca gagcgaggta 3720

tgtaggcggt gctacagagt tcttgaagtg gtggcctaac tacggctaca ctagaagaac 3780

agtatttggt atctgcgctc tgctgtagcc agttaccttc ggaaaaagag ttggtagctc 3840

ttgatccggc aaacaaacca ccgctggtag cggtggtttt tttgtttgca agcagcagat 3900

tacgcgcaga aaaaaaggat ctcaagaaga tcctttgatc ttttctacgg ggtctgacgc 3960

tcagtggaac gaaaactcac gttaagggat tttggtcaga tctagcacca ggcgtttaag 4020

ggcaccaata actgccttaa aaaaattacg ccccgccctg ccactcatcg cagtactgtt 4080

gtaattcatt aagcattctg ccgacatgga agccatcaca aacggcatga tgaacctgaa 4140

tcgccagcgg catcagcacc ttgtcgcctt gcgtataata tttgcccata gtgaaaacgg 4200

gggcgaagaa gttgtccata ttggctacgt ttaaatcaaa actggtgaaa ctcacccagg 4260

gattggctga gacgaaaaac atattctcaa taaacccttt agggaaatag gccaggtttt 4320

caccgtaaca cgccacatct tgcgaatata tgtgtagaaa ctgccggaaa tcgtcgtggt 4380

attcactcca gagcgatgaa aacgtttcag tttgctcatg gaaaacggtg taacaagggt 4440

gaacactatc ccatatcacc agctcaccgt ctttcattgc catacggaac tccgggtgag 4500

cattcatcag gcgggcaaga atgtgaataa aggccggata aaacttgtgc ttatttttct 4560

ttacggtctt taaaaaggcc gtaatatcca gctgaacggt ctggttatag gtacattgag 4620

caactgactg aaatgcctca aaatgttctt tacgatgcca ttgggatata tcaacggtgg 4680

tatatccagt gatttttttc tccattttag cttccttagc tcctgaaaat ctcgataact 4740

caaaaaatac gcccggtagt gatcttattt cattatggtg aaagttggaa cctcacccga 4800

cgtctaatgt gagttagctc actcattagg caccccaggc tttacacttt atgcttccgg 4860

ctcgtatgtt gtgtggaatt gtgagcggat aacaatttca cacaggaaac agctatgacc 4920

atgattacga atttctagat aacgagggca aaaaatgaaa aagacagcta tcgcgattgc 4980

agtggcactg gctggtttcg ctaccgtagc gcaggccgat 5020

<210> 37
<211> 4145
<212> DNA
<213> artificial sequence

<220>
<221> pMORPH18_Fab_GPC8
<222> (1)..(4145)

<400> 37
tcagataacg agggcaaaaa atgaaaaaga cagctatcgc gattgcagtg gcactggctg 60

gtttcgctac cgtagcgcag gccgatatcg tgctgaccca gccgccttca gtgagtggcg 120

caccaggtca gcgtgtgacc atctcgtgta gcggcagcag cagcaacatt ggcagcaact 180

atgtgagctg gtaccagcag ttgcccggga cggcgccgaa actgctgatt tatgataaca 240

accagcgtcc ctcaggcgtg ccggatcgtt ttagcggatc caaaagcggc accagcgcga 300

gccttgcgat tacgggcctg caaagcgaag acgaagcgga ttattattgc cagagctatg 360

acatgcctca ggctgtgttt ggcggcggca cgaagtttaa ccgttcttgg ccagccgaaa 420

gccgcaccga gtgtgacgct gtttccgccg agcagcgaag aattgcaggc gaacaaagcg 480

accctggtgt gcctgattag cgacttttat ccgggagccg tgacagtggc ctggaaggca 540

gatagcagcc ccgtcaaggc gggagtggag accaccacac cctccaaaca aagcaacaac 600

aagtacgcgg ccagcagcta tctgagcctg acgcctgagc agtggaagtc ccacagaagc 660

tacagctgcc aggtcacgca tgaggggagc accgtggaaa aaaccgttgc gccgactgag 720

gcctgataag catgcgtagg agaaaataaa atgaaacaaa gcactattgc actggcactc 780

ttaccgttgc tcttcacccc tgttaccaaa gcccaggtgc aattgaaaga aagcggcccg 840

gccctggtga aaccgaccca aaccctgacc ctgacctgta ccttttccgg atttagcctg 900

tccacgtctg gcgttggcgt gggctggatt cgccagccgc ctgggaaagc cctcgagtgg 960

ctggctctga ttgattggga tgatgataag tattatagca ccagcctgaa aacgcgtctg 1020

accattagca aagatacttc gaaaaatcag gtggtgctga ctatgaccaa catggacccg 1080

gtggatacgg ccacctatta ttgcgcgcgt tctcctcgtt atcgtggtgc ttttgattat 1140

tggggccaag gcaccctggt gacggttagc tcagcgtcga ccaaaggtcc aagcgtgttt 1200

ccgctggctc cgagcagcaa aagcaccagc ggcggcacgg ctgccctggg ctgcctggtt 1260

aaagattatt tcccggaacc agtcaccgtg agctggaaca gcggggcgct gaccagcggc 1320

gtgcatacct ttccggcggt gctgcaaagc agcggcctgt atagcctgag cagcgttgtg 1380

accgtgccga gcagcagctt aggcactcag acctatattt gcaacgtgaa ccataaaccg 1440

agcaacacca aagtggataa aaaagtggaa ccgaaaagcg aattcggggg agggagcggg 1500

agcggtgatt ttgattatga aaagatggca aacgctaata agggggctat gaccgaaaat 1560

gccgatgaaa acgcgctaca gtctgacgct aaaggcaaac ttgattctgt cgctactgat 1620

tacggtgctg ctatcgatgg tttcattggt gacgtttccg gccttgctaa tggtaatggt 1680

gctactggtg attttgctgg ctctaattcc caaatggctc aagtcggtga cggtgataat 1740

tcacctttaa tgaataattt ccgtcaatat ttaccttccc tccctcaatc ggttgaatgt 1800

cgcccttttg tctttggcgc tggtaaacca tatgaatttt ctattgattg tgacaaaata 1860

aacttattcc gtggtgtctt tgcgtttctt ttatatgttg ccacctttat gtatgtattt 1920

tctacgtttg ctaacatact gcgtaataag gagtcttgat aagcttgacc tgtgaagtga 1980

aaaatggcgc agattgtgcg acattttttt tgtctgccgt ttaatgaaat tgtaaacgtt 2040

aatattttgt taaaattcgc gttaaatttt tgttaaatca gctcattttt taaccaatag 2100

gccgaaatcg gcaaaatccc ttataaatca aaagaataga ccgagatagg gttgagtgtt 2160

gttccagttt ggaacaagag tccactatta aagaacgtgg actccaacgt caaagggcga 2220

aaaaccgtct atcagggcga tggcccacta cgagaaccat caccctaatc aagttttttg 2280

gggtcgaggt gccgtaaagc actaaatcgg aaccctaaag ggagcccccg atttagagct 2340

tgacggggaa agccggcgaa cgtggcgaga aaggaaggga agaaagcgaa aggagcgggc 2400

gctagggcgc tggcaagtgt agcggtcacg ctgcgcgtaa ccaccacacc cgccgcgctt 2460

aatgcgccgc tacagggcgc gtgctagcca tgtgagcaaa aggccagcaa aaggccagga 2520

accgtaaaaa ggccgcgttg ctggcgtttt tccataggct ccgcccccct gacgagcatc 2580

acaaaaatcg acgctcaagt cagaggtggc gaaacccgac aggactataa agataccagg 2640

cgtttccccc tggaagctcc ctcgtgcgct ctcctgttcc gaccctgccg cttaccggat 2700

acctgtccgc ctttctccct tcgggaagcg tggcgctttc tcatagctca cgctgtaggt 2760

atctcagttc ggtgtaggtc gttcgctcca agctgggctg tgtgcacgaa ccccccgttc 2820

agtccgaccg ctgcgcctta tccggtaact atcgtcttga gtccaacccg gtaagacacg 2880

acttatcgcc actggcagca gccactggta acaggattag cagagcgagg tatgtaggcg 2940

gtgctacaga gttcttgaag tggtggccta actacggcta cactagaaga acagtatttg 3000

gtatctgcgc tctgctgtag ccagttacct tcggaaaaag agttggtagc tcttgatccg 3060

gcaaacaaac caccgctggt agcggtggtt tttttgtttg caagcagcag attacgcgca 3120

gaaaaaaagg atctcaagaa gatcctttga tcttttctac ggggtctgac gctcagtgga 3180

acgaaaactc acgttaaggg attttggtca gatctagcac caggcgttta agggcaccaa 3240

taactgcctt aaaaaaatta cgccccgccc tgccactcat cgcagtactg ttgtaattca 3300

ttaagcattc tgccgacatg gaagccatca caaacggcat gatgaacctg aatcgccagc 3360

ggcatcagca ccttgtcgcc ttgcgtataa tatttgccca tagtgaaaac gggggcgaag 3420

aagttgtcca tattggctac gtttaaatca aaactggtga aactcaccca gggattggct 3480

gagacgaaaa acatattctc aataaaccct ttagggaaat aggccaggtt ttcaccgtaa 3540

cacgccacat cttgcgaata tatgtgtaga aactgccgga aatcgtcgtg gtattcactc 3600

cagagcgatg aaaacgtttc agtttgctca tggaaaacgg tgtaacaagg gtgaacacta 3660

tcccatatca ccagctcacc gtctttcatt gccatacgga actccgggtg agcattcatc 3720

aggcgggcaa gaatgtgaat aaaggccgga taaaacttgt gcttattttt ctttacggtc 3780

tttaaaaagg ccgtaatatc cagctgaacg gtctggttat aggtacattg agcaactgac 3840

tgaaatgcct caaaatgttc tttacgatgc cattgggata tatcaacggt ggtatatcca 3900

gtgatttttt tctccatttt agcttcctta gctcctgaaa atctcgataa ctcaaaaaat 3960

acgcccggta gtgatcttat ttcattatgg tgaaagttgg aacctcaccc gacgtctaat 4020

gtgagttagc tcactcatta ggcaccccag gctttacact ttatgcttcc ggctcgtatg 4080

ttgtgtggaa ttgtgagcgg ataacaattt cacacaggaa acagctatga ccatgattac 4140

gaatt 4145

<210> 38
<211> 120
<212> PRT
<213> artificial sequence

<220>
<221> MS-GPC1-VH
<222> (1)..(120)

<400> 38

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Gln Tyr Gly His Arg Gly Gly Phe Asp His Trp Gly Gln
            100                 105                 110

Gly Thr Leu Val Thr Val Ser Ser
        115                 120

<210> 39
<211> 109
<212> PRT
<213> artificial sequence

<220>
<221> MS-GPC1-VL
<222> (1)..(109)

<400> 39

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Phe Asn Glu
                85                  90                  95

Ser Val Phe Gly Gly Gly Thr Lys Leu Thr Val Leu Gly
            100                 105

<210> 40
<211> 131
<212> PRT
<213> artificial sequence

<220>
<221> MS-GPC2-VH
<222> (1)..(131)

<400> 40

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Gly Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ser His Asn Lys Lys Trp Arg Phe Tyr Asn Leu
            100                 105                 110

Tyr Ser Leu Tyr Asp Phe Asp Phe Trp Gly Gln Gly Thr Leu Val Thr
        115                 120                 125

Val Ser Ser
    130

<210>  41
<211>  110
<212>  PRT
<213>  artificial sequence

<220>
<221>  MS-GPC2-VL
<222>  (1)..(110)

<400>  41

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Glu Ser Gly Phe Pro
                85                  90                  95

Tyr Thr Phe Gly Gln Gly Thr Lys Val Glu Ile Lys Arg Thr
            100                 105                 110

<210>  42
<211>  116
<212>  PRT
<213>  artificial sequence

<220>
<221>  MS-GPC3-VH
<222>  (1)..(116)

<400>  42

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Leu Ser Thr Arg Met Asp Pro Trp Gly Gln Gly Thr Leu Val
            100                 105                 110

Thr Val Ser Ser
        115

<210>  43
<211>  110
<212>  PRT
<213>  artificial sequence

<220>
<221>  MS-GPC3-VL
<222>  (1)..(110)

<400>  43

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Asp Asp Asn Phe Pro
                85                  90                  95

Ile Thr Phe Gly Gln Gly Thr Lys Val Glu Ile Lys Arg Thr
            100                 105                 110

<210>  44
<211>  126
<212>  PRT
<213>  artificial sequence

<220>
<221>  MS-GPC4-VH
<222>  (1)..(126)

<400>  44

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Tyr Tyr Val Tyr Ser Val Gly Tyr Gly Val Thr His Tyr
            100                 105                 110

Asp Asp Val Trp Gly Gln Gly Thr Leu Val Thr Val Ser Ser
        115                 120                 125

<210>  45
<211>  110
<212>  PRT
<213>  artificial sequence

<220>
<221>  MS-GPC4-VL
<222>  (1)..(110)

<400>  45

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Asp Tyr Ser Tyr Pro
                85                  90                  95

Ser Thr Phe Gly Gln Gly Thr Lys Val Glu Ile Lys Arg Thr
            100                 105                 110

<210>  46
<211>  115
<212>  PRT
<213>  artificial sequence

<220>
<221>  MS-GPC5-VH
<222>  (1)..(115)

<400>  46

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg His Ser Phe Phe Asp Tyr Trp Gly Gln Gly Thr Leu Val Thr
            100                 105                 110

Val Ser Ser
        115

<210>  47
<211>  108
<212>  PRT
<213>  artificial sequence

<220>
<221>  MS-GPC5-VL
<222>  (1)..(108)

<400>  47

Asp Ile Glu Leu Thr Gln Pro Pro Ser Val Ser Val Ala Pro Gly Gln
1               5                   10                  15

Thr Ala Arg Ile Ser Cys Ser Gly Asp Ala Leu Gly Asp Lys Tyr Ala
            20                  25                  30

Ser Trp Tyr Gln Gln Lys Pro Gly Gln Ala Pro Val Leu Val Ile Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Ser Tyr Asp Asn Val Asp Ile Ser
                85                  90                  95

Val Phe Gly Gly Gly Thr Lys Leu Thr Val Leu Gly
            100                 105

<210>  48
<211>  118
<212>  PRT
<213>  artificial sequence

<220>
<221>  MS-GPC6-VH
<222>  (1)..(118)

<400>  48

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Tyr Gly Arg Tyr Ser Pro Asp Leu Trp Gly Gln Gly Thr
            100                 105                 110

Leu Val Thr Val Ser Ser
        115

<210>  49
<211>  110
<212>  PRT
<213>  artificial sequence

<220>
<221>  MS-GPC6-VL
<222>  (1)..(110)

<400>  49

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Tyr Ser Asn Leu Pro
                85                  90                  95

Phe Thr Phe Gly Gln Gly Thr Lys Val Glu Ile Lys Arg Thr
            100                 105                 110

<210>  50

<211>  122
<212>  PRT
<213>  artificial sequence

<220>
<221>  MS-GPC7-VH
<222>  (1)..(122)

<400>  50

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Ser Gln Asn Gly Phe Tyr Gly Gly Asn Leu Asp Ile Trp
            100                 105                 110

Gly Gln Gly Thr Leu Val Thr Val Ser Ser
        115                 120

<210>  51
<211>  109
<212>  PRT
<213>  artificial sequence

<220>
<221>  MS-GPC7-VL
<222>  (1)..(109)

<400>  51

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Arg Asp Pro Ser Asn
                85                  90                  95

Val Val Phe Gly Gly Gly Thr Lys Leu Thr Val Leu Gly
            100                 105

<210> 52
<211> 120
<212> PRT
<213> artificial sequence

<220>
<221> MS-GPC8-VH
<222> (1)..(120)

<400> 52

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Ser Pro Arg Tyr Arg Gly Ala Phe Asp Tyr Trp Gly Gln
            100                 105                 110

Gly Thr Leu Val Thr Val Ser Ser
        115                 120

<210> 53
<211> 109
<212> PRT
<213> artificial sequence

<220>
<221> MS-GPC8-VL
<222> (1)..(109)

<400> 53

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Met Pro Gln
                85                  90                  95

Ala Val Phe Gly Gly Gly Thr Lys Leu Thr Val Leu Gly
            100                 105

<210>  54
<211>  120
<212>  PRT
<213>  artificial sequence

<220>
<221>  MS-GPC10-VH
<222>  (1)..(120)

<400>  54

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Gln Leu His Tyr Arg Gly Gly Phe Asp Leu Trp Gly Gln
            100                 105                 110

Gly Thr Leu Val Thr Val Ser Ser
        115                 120

<210>  55
<211>  109
<212>  PRT
<213>  artificial sequence

<220>
<221>  MS-GPC10-VL
<222>  (1)..(109)

<400>  55

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Leu Thr Met
                85                  90                  95

Gly Val Phe Gly Gly Gly Thr Lys Leu Thr Val Leu Gly
            100                 105

<210>  56
<211>  120
<212>  PRT
<213>  artificial sequence

<220>
<221>  MS-GPC11-VH
<222>  (1)..(120)

<400>  56

Gln Val Gln Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Asp Trp Asp Asp Asp Lys Tyr Tyr Ser Thr Ser
    50                  55                  60

Leu Lys Thr Arg Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Ser Gln Gly Tyr Arg Gly Gly Leu Asp Val Trp Gly Gln
            100                 105                 110

Gly Thr Leu Val Thr Val Ser Ser
        115                 120

<210>  57
<211>  109
<212>  PRT
<213>  artificial sequence

<220>
<221>  MS-GPC11-VL
<222>  (1)..(109)

<400>  57

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Tyr Gly Ile
                85                  90                  95

Tyr Val Phe Gly Gly Gly Thr Lys Leu Thr Val Leu Gly
            100                 105

<210> 58
<211> 121
<212> PRT
<213> artificial sequence

<220>
<221> MS-GPC14-VH
<222> (1)..(121)

<400> 58

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Ser Met Pro Met Tyr Gly Glu Gly Phe Asp Leu Trp Gly
            100                 105                 110

Gln Gly Thr Leu Val Thr Val Ser Ser
        115                 120

<210> 59
<211> 109
<212> PRT
<213> artificial sequence

<220>
<221> MS-GPC14-VL
<222> (1)..(109)

<400> 59

Asp Ile Glu Leu Thr Gln Pro Pro Ser Val Ser Val Ala Pro Gly Gln
1               5                   10                  15

Thr Ala Arg Ile Ser Cys Ser Gly Asp Ala Leu Gly Asp Lys Tyr Ala
            20                  25                  30

Ser Trp Tyr Gln Gln Lys Pro Gly Gln Ala Pro Val Leu Val Ile Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Ser Tyr Asp Phe Gly Val Ser His
                85                  90                  95

Ser Val Phe Gly Gly Gly Thr Lys Leu Thr Val Leu Gly
            100                 105

<210>  60
<211>  120
<212>  PRT
<213>  artificial sequence

<220>
<221>  MS-GPC15-VH
<222>  (1)..(120)

<400>  60

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Phe Tyr Tyr Ser His Val Leu Ala Met Asp Asn Trp Gly Gln
            100                 105                 110

Gly Thr Leu Val Thr Val Ser Ser
        115                 120

<210>  61
<211>  109
<212>  PRT
<213>  artificial sequence

<220>
<221>  MS-GPC15-VL

<222>  (1)..(109)

<400>  61

Asp Ile Glu Leu Thr Gln Pro Pro Ser Val Ser Val Ala Pro Gly Gln
1               5                   10                  15

Thr Ala Arg Ile Ser Cys Ser Gly Asp Ala Leu Gly Asp Lys Tyr Ala
            20                  25                  30

Ser Trp Tyr Gln Gln Lys Pro Gly Gln Ala Pro Val Leu Val Ile Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Glu
65                  70                  75                  80

Asp Glu Ala Asp Tyr Tyr Cys Gln Ser Arg Asp Ile His Ile His Asn
                85                  90                  95

Glu Val Phe Gly Gly Gly Thr Lys Leu Thr Val Leu Gly
            100                 105

<210>  62
<211>  120
<212>  PRT
<213>  artificial sequence

<220>
<221>  MS-GPC16-VH
<222>  (1)..(120)

<400>  62

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Gly Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Thr Gln Leu Tyr Tyr Phe Asp Tyr Trp Gly Gln
            100                 105                 110

Gly Thr Leu Val Thr Val Ser Ser
        115                 120

<210>  63
<211>  114
<212>  PRT

<213>  artificial sequence

<220>
<221>  MS-GPC16-VL
<222>  (1)..(114)

<400>  63

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gln Gln Tyr
                85                  90                  95

Asn Ser Tyr Pro Arg Thr Phe Gly Gln Gly Thr Lys Val Glu Ile Lys
            100                 105                 110

Arg Thr


<210>  64
<211>  109
<212>  PRT
<213>  artificial sequence

<220>
<221>  MS-GPC8-6-VL
<222>  (1)..(109)

<400>  64

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Tyr Asp His
                85                  90                  95

Tyr Val Phe Gly Gly Gly Thr Lys Leu Thr Val Leu Gly
            100                 105

<210>  65
<211>  109
<212>  PRT
<213>  artificial sequence

<220>
<221>  MS-GPC8-17-VL
<222>  (1)..(109)

<400>  65

Asp Ile Val Leu Thr Gln Pro Pro Ser Val Ser Gly Ala Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asn Asn Gln Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ser Glu Asp Glu Ala Asp Tyr Tyr Cys Gln Ser Tyr Asp Phe Ser Val
                85                  90                  95

Tyr Val Phe Gly Gly Gly Thr Lys Leu Thr Val Leu Gly
            100                 105

## Description

The present invention relates to human peptides/proteins causing or leading to the modulation of the immune system. The invention further relates to nucleic acids encoding the peptides/proteins, methods for production of the peptides/proteins, methods for immunosuppression, pharmaceutical and diagnostic compositions and kits comprising the peptides/proteins and uses of the peptides/proteins.

Diseases involving the immune system are significantly debilitating to suffering individuals and are predicted to increase in number of patients over the next 10 years. Such diseases include rheumatoid arthritis (RA), multiple sclerosis (MS), type I diabetes, transplant rejection (TR) and graft vs. host disease (GvHD). The recorded number of patients for these diseases for 1995, the predicted number of patients for 2010 and corresponding market sizes are shown in Table 1. The number of patients suffering from rheumatoid arthritis for example is expected to grow world-wide from 6.6 million to 7 million by 2010.

**Table 1**

| | Number of patients (Mio) | | Market size (Bio.$) | |
|---|---|---|---|---|
| Disease | 1995 | 2010 | 1994 | 2010(est) |
| Rheumatoid Arthritis | 6.6 | 7 | 2.4 | >3.7 |
| Multiple Sclerosis | 0.62 | 0.65 | 0.3 | >1.5 |
| Type I diabetes | 1.8 | 1.9 | 1.5 | >1.5 |
| Transplant/GvHD | 0.05 | 0.1 | 0.9 | >1.5 |

Current therapies in diseases of the immune system include anti-inflammatory drugs, e.g., NSAIDS (non-steroidal anti-inflammatory drugs), corticosteroids, cytostatics (methotrexate for RA), and cytokines (interferon beta for MS). These therapies are symptomatic; none of them induces complete remission of the disease. A general problem with most current drugs is also their lack of selectivity: they suppress the whole immune system, and thus, the patients treated will become highly susceptible to infections. Finally, the side effect profile of most presently used anti-inflammatory agents also warrants the development of new therapeutics for these diseases. Therefore, there is a pressing unmet medical need for selective, disease-mechanism-based therapeutics to treat diseases of the immune system such as RA and MS.

The underlying immunological mechanisms of TR and GvHD are similar to those in diseases of the immune system. In organ transplantation, the recipient's immune system attacks the foreign organ, whereas in GvHD the foreign hematopoietic cells introduced into immunocomprimised hosts attack the host. At present, corticosteroids, Azathioprine, Cyclosporin A, and CellCept are used for prevention of rejection, and high dose corticosteroids, OKT3 (a monoclonal antibody (mAb) to a pan-T cell marker), and Zenapax (mAb to IL-2R on activated T cells) for its treatment. In GvHD corticosteroids are used, but no satisfactory treatment is available. There is an unmet medical need for a better tolerated and more effective immunosuppressant, particularly for the treatment of GvHD.

Every mammalian species, which has been studied to date carries a cluster of genes coding for the so called major histocompatibility complex (MHC). This tightly linked cluster of genes code for surface antigens, which play a central role in the development of both humoral and cell-mediated immune responses. In humans the products coded for by the MHC are referred to as Human Leukocyte Antigens or HLA. The MHC-genes are organised into regions encoding three classes of molecules, class I to III.

Class I MHC molecules are 45 kD transmembrane glycoproteins, noncovalently associated with another glycoprotein, the 12 kD beta-2 microglobulin (Brown et al., 1993). The latter is not inserted into the cell membrane, and is encoded outside the MHC. Human class I molecules are of three different isotypes, termed HLA-A, -B, and -C, encoded in separate loci. The tissue expression of class I molecules is ubiquitous and codominant. MHC class I molecules present peptide antigens necessary for the activation of cytotoxic T-cells.

Class II MHC molecules are noncovalently associated heterodimers of two transmembrane glycoproteins, the 35 kD α chain and the 28 kD β chain (Brown et al., 1993). In humans, class II molecules occur as three different isotypes, termed human leukocyte antigen DR (HLA-DR), HLA-DP and HLA-DQ. Polymorphism in DR is restricted to the β chain, whereas both chains are polymorphic in the DP and DQ isotypes. Class II molecules are expressed codominantly, but in contrast to class I, exhibit a restricted tissue distribution: they are present only on the surface of cells of the immune system, for example dendritic cells, macrophages, B lymphocytes, and activated T lymphocytes. They are also expressed on human adrenocortical cells in the zona reticularis of normal adrenal glands and on granulosa-lutein cells in corpora lutea of normal ovaries (Kahoury et al., 1990). Their major biological role is to bind antigenic peptides and present them on the surface of antigen presenting cells (APC) for recognition by CD4 helper T (Th) lymphocytes (Babbitt et al., 1985.) MHC class II molecules can also be expressed on the surface of non-immune system cells. For example, cells in an organ other than lymphoid cells can express MHC class II molecules during a pathological inflammatory response. These cells may include synovial cells, endothelial cells, thyroid stromal cells and glial cells.

Class III MHC molecules are also associated with immune responses, but encode somewhat different products. These include a number of soluble serum proteins, enzymes and proteins like tumour necrosis factor or steroid 21-hydroxylase enzymes. In humans, class III molecules occur as three different isotypes, termed Ca, C2 and Bf (Kuby, 1994).

A large body of evidence has demonstrated that susceptibility to many diseases, in particular diseases of the immune system, is strongly associated with specific alleles of the major histocompatibility complex (reviewed in Tiwari et al., 1985). Although some class I associated diseases exist, most autoimmune conditions have been found to be associated with class II alleles. For example, class II alleles DRB1*0101, 0401, 0404, and 0405 occur at increased frequency among rheumatoid arthritis (RA) patients (McMichael et al., 1977; Stasny, 1978; Ohta et al., 1982; Schiff et al., 1982), whereas DRB1*1501 is associated with multiple sclerosis (MS), and the DQ allele combination DQA1*0301/B1*0302 with insulin dependent diabetes mellitus (IDDM). In RA, altogether >94% of rheumatoid factor positive patients carry one of the susceptibility alleles (Nepom et al., 1989).

Class II MHC molecules are the primary targets for immunosuppressive intervention for the following reasons: First, MHC-II molecules activate T helper (Th) cells that are central to immunoregulation, and are responsible for most of the immunopathology in inflammatory diseases. Second, most diseases of the immune system are genetically associated with class II alleles. Third, MHC-II molecules are only expressed on cells of the immune system, whereas MHC-I molecules are present on most somatic cells.

At least three mechanisms are believed to play some part in immunosuppression mediated by proteins binding to MHC class II molecules. First, since Th cells recognise antigenic peptides bound to class II molecules, monoclonal antibodies (mAb) specific for class II molecules can sterically hinder the interaction between the MHC class II molecule and the T cell receptor, and thereby prevent Th cell activation. Indeed, this has been shown to occur both in vitro and in vivo (Baxevanis et al., 1980; Nepom et al., 1981; Rosenbaum et al., 1983). Second, down regulation of cell surface expression of MHC class II molecules has been shown to associate with immunosuppression using certain mouse anti-MHC class II antibodies (Vidovic et al., 1995). Third, killing of activated lymphoid cells occurs when certain anti-MHC class II antibodies bind to antigen expressed on the surface of these cells (Vidovic et al., 1995a). Increased selectivity of treatment is achieved since only cells expressing the specific MHC class II antigen can be targeted by a specific monoclonal antibody and hence only the immune response mediated by these allotypes is modulated. Host-defence immune reactions which are mediated by other MHC molecules are not targeted by the specific antibody and hence remain unmodulated and non-comprimised.

Based on these observations, anti-class II mAb have been envisaged for a number of years as therapeutic candidates for the immunosuppressive treatment of disorders of the immune system includingtransplant rejection. Indeed, this hypothesis has been supported by the beneficial effect of mouse-derived anti-class II mAbs in a series of animal disease models (Waldor et al., 1983; Jonker et al., 1988; Stevens et al., 1990; Smith et al., 1994).

Despite these early supporting data, to date no anti-MHC class II mAb of human composition has been described that displays the desired immunomodulatory and other biological properties. Indeed, despite the relative ease by which mouse-derived mAbs may be derived, work using mouse-derived mAbs has demonstrated the difficulty of obtaining an immunomodulatory antibody with the desired biological properties. For example, significant and not fully understood differences were observed in the T cell inhibitory capacity of different anti-class II mAbs (Naquet et al., 1983). Furthermore, the application of certain mouse-derived mAbs *in vivo* was associated with unexpected side effects, sometimes resulting in death of laboratory primates (Billing et al., 1983; Jonker et al., 1991).

It is generally accepted that mouse-derived mAbs (including chimeric and so-called 'humanised' mAbs) carry an increased risk of generating an adverse immune response in patients compared to treatment with a human mAb. This risk is potentially increased when treating chronic diseases such as RA or MS with any mouse-derived mAb; prolonged exposure of the human immune system to a non-human molecule often leads to the development of an adverse immune reaction. Furthermore, it is significantly difficult to obtain mouse-derived antibodies with the desired specificity or affinity to the desired antigen. Such observation may have a significant influence on the overall therapeutic effect or advantage provided by mouse-derived mAbs. Finally, even if a mouse mAb could be identified that displayed the desired specificity or affinity, often these desired features are detrimentally affected during the 'humanisation' or 'chimerisation' procedures necessary to reduce immunogenic potential. Once a mouse-derived mAb has been 'humanised' or 'chimerised', then it is significantly difficult to optimise its specificity or affinity.

The art has sought over a number of years for anti-MHC class II mAbs of human composition that show immunomodulatory and other biological properties suitable for use in a pharmaceutical composition for the treatment of humans. Workers in the field have practised the process steps of first identifying a mouse-derived mAb, and then modifying the structure of this mAb with the aim of improving immunotolerance of this non-human molecule for human patients (for further details, see Jones et al., 1986; Riechmann et al., 1988; Presta, 1992). This modification is typically made using so-called 'humanisation' procedures or by fabricating a human-mouse chimeric mAb. Other workers have attempted to identify human antibodies that bind to human antigens having desired properties within natural repertoires of human antibody diversity. For example, by exploring the foetal-tolerance mechanism in pregnant women (Bonagura et al.,1987) or by panning libraries of natural diversities of antibodies (Stausbøl-Grøn et al., 1996; Winter et al., 1994). However, to date no anti-MHC class II mAb of human composition has been described that displays the desired biological properties of immunomodulation, specificity, low immunogenicity and affinity.

For therapeutic purposes a peptide/protein reacting with most or all allelic forms of a human class II MHC molecule would be desirable. Moreover, the candidate peptide/protein should trigger an immuno-modulatory mechanism applicable to a wide range of diseases of the immune system or transplantation associated complications.

Thus, the technical problem underlying the present invention is to provide means causing or leading to an immunomodulatory effect for therapeutic purposes with a minimum of non-desired side effects.

The solution to the above technical problems is achieved by the embodiments characterised in the claims. In particular, the above problem is solved by a peptide/protein comprising at least one antibody-based antigen-binding domain of human composition with binding specificity for a human MHC class II antigen, wherein binding of one or more of said peptides/proteins to cells expressing said antigen causes or leads to the modulation of the immune response of a subject treated with said peptide/proteins.

### Definitions

As used herein, the term "peptide" relates to molecules consisting of one or more chains of multiple, i. e. two or more, amino acids linked via peptide bonds. The term "protein" refers to peptides where at least part of the peptide has or is able to acquire a defined three-dimensional arrangement by forming secondary, tertiary, or quaternary structures within and/or between its peptide chain(s). This definition comprises proteins such as naturally occurring or at least partially artificial proteins, as well as fragments or domains of whole proteins, as long as these fragments or domains are able to acquire a defined three-dimensional arrangement as described above.

In this context, "peptide/protein comprising at least one antibody-based antigen-binding domain" refers to an immunoglobulin (e.g. IgG, IgA or IgM molecules or antibody) or to a functional fragment thereof. The term "functional fragment" refers to a fragment of an immunoglobulin which retains the antigen-binding moiety of an immunoglobulin. Functional immunoglobulin fragments according to the present invention may be Fv (Skerra and Plückthun, 1988), scFv (Bird et al., 1988; Huston et al., 1988), disulfide-linked Fv (Glockshuber et al., 1992; Brinkmann et al., 1993), Fab, F(ab')₂ fragments or other fragments well-known to the practitioner skilled in the art, which comprise the variable domains of an immunoglobulin or functional immunoglobulin fragment.

Examples of peptides/proteins consisting of one chain are single-chain Fv antibody fragments, and examples for peptides/proteins consisting of more chains are Fab antibody fragments.

As used herein, an "antibody-based antigen-binding domain of human composition" means a peptide/protein comprising at least an antibody VH domain and an antibody VL domain, wherein a homology search in a database of protein sequences comprising immunoglobulin sequences results for both the VH and the VL domain in an immunoglobulin domain of human origin as hit with the highest degree of sequence identity. Such a homology search may be a BLAST search, e.g. by accessing http://www.ncbi.nlm.nih.gov/BLAST and performing a "BasicBLAST" search using the "blastp" routine. Preferably, such a composition does not elicit an adverse immune response thereto when administered to a human recipient.

Furthermore the term "functional fragment" may refer to a "multivalent composition", which means a composition comprising at least two of said antigen-binding domains. Preferably, said at least two antigen-binding domains are in close proximity so as to mimic the structural arrangement relative to each other of binding sites comprised in a full immunoglobulin molecule. Examples for multivalent compositions are full immunoglobulin molecules, multivalent fragments thereof (e.g. F(ab')₂) or full immunoglobulin molecules that are cross-linked. Multivalent compositions, however, may be formed as well from two or more monovalent immunoglobulin fragments, e.g. by self-association as in mini-antibodies, or by cross-linking.

As used herein, the term "mini-antibody fragment" means a multivalent antibody fragment comprising at least two antigen-binding domains multimerized by self-associating domains fused to each of said domains (Pack, 1994), e.g. dimers comprising two scFv fragments, each fused to a self-associating dimerization domain. Dimerization domains, which are particularly preferred, include those derived from a leucine zipper (Pack and Plückthun, 1992) or helix-turn-helix motif (Pack et al., 1993).

As used herein, "activated cells" means cells of a certain population of interest, which are not resting. Activation might be caused by antigens, mitogens (e.g., lipopoysaccharide, phytohemagglutinine) or cytokines (e.g., interferon gamma). Preferably, said activation occurs during the stimulation of resting T and B cells in the course of the generation of an immune response. Activated cells may be certain lymphoid tumour cells. Preferably, activated cells are characterised by the feature of MHC class II molecules expressed on the cell surface and one or more additional feature including increased cell size, cell division, DNA replication, expression of CD45 or CD11 and production/secretion of immunoglobulin.

As used herein, "non-activated cells" means cells of a population of interest, the vast majority of which are resting and non-dividing. Said non-activated cells may include resting B cells as purified from healthy human blood. Such cells can, preferably, be characterised by lack or reduced level of MHC class II molecules expressed on the cell surface and lack or reduced level of one or more additional features including increased cell size, cell division, DNA replication, expression of CD45 or CD11 and production/secretion of immunoglobulin.

"Lymphoid cells" when used in reference to a cell line or a cell, means that the cell line or cell is derived from the lymphoid lineage and includes cells of both the B and the T lymphocyte lineages, and the macrophage lineage.

"Non lymphoid cells and express MHC class II" means cells other than lymphoid cells that express MHC class II molecules during a pathological inflammatory response. For example, said cells may include synovial cells, endothelial cells, thyroid stromal cells and glial cells and it may also comprise genetically altered cells capable of expressing MHC-class II molecules.

As used herein, the term "first domain of the alpha-chain of HLA-DR" means the N-terminal domain of the alpha-chain.

As used herein, the term "first domain of the beta-chain of HLA-DR" means the N-terminal domain of the beta-chain.

As used herein, the term "modulation of the immune response" relates to the changes in activity of the immune response of an individual or to changes of an *in vitro* system resembling parts of an immune system. Said changes in activity are causing or leading to immunosuppression.

The terms "apoptosis" and "apoptotic activity" refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. Apoptosis follows a very stringent time course and is executed by caspases, a specific group of proteases. Apoptotic activity can be determined and measured, for instance, by cell viability assays, Annexin V staining or caspase inhibition assays. Apoptosis can be induced using a cross-linking antibody such as anti-CD95 as described in Example G.

The term "innate pre-programmed process" refers to a process that, once it is started, follows an autonomous cascade of mechanisms within a cell, which does not require any further auxiliary support from the environment of said cell in order to complete the process.

As used herein, the term "HuCAL" refers to a fully synthetic human combinatorial antibody library as described in Knappik et al. (2000).

As used herein, the term "CDR3" refers to the third complementarity-determining region of the VH and VL domains of antibodies or fragments thereof, wherein the VH CDR3 covers positions 95 to 102 (possible insertions after positions 100 listed as 100a to 100z), and VL CDR3 positions 89 to 96 (possible insertions in Vλ after position 95 listed as 95a to 95c) (see Knappik et al., 2000).

As used herein, the term "hybridises under stringent conditions" is intended to describe conditions for hybridisation and washing under which nucleotide sequences at least 60% homologous to each other typically remain hybridised to each other. Preferably, the conditions are such that at least sequences at least 65%, more preferably at least 70%, and even more preferably at least 75% homologous to each other typically remain hybridised to each other. Such stringent conditions are known to those skilled in the art and can be found in Current Protocols in Molecular Biology, John Wiley & Sons, New York (1999), 6.3.1-6.3.6. A preferred, non-limiting example of stringent hybridisation conditions is hybridisation in 6x sodium chloride/sodium citrate (SSC) at about 45°C, followed by one or more washes in 0.2x SSC, 0.1% SDS at 50°-65°C.

The peptide/protein according to the invention is comprising at least one antibody-based antigen-binding domain of human composition with binding specificity for a human MHC class II antigen, wherein binding of said peptide/protein to said antigen expressed on the surface of a cell causes or leads to a modulation of the immune response.

According to a preferred embodiment, the peptide/protein is directed to a lymphoid cell or a non-lymphoid cell that expresses MHC class II molecules. The latter type of cells occurs for example at pathological sites of inflammation and/or diseases of the immune system. Said cells may include synovial cells, endothelial cells, thyroid stromal cells and glial cells.

In a further preferred embodiment the peptide/protein binds to an antigen comprising epitopes selected from HLA-DR molecules.

In a further preferred embodiment the peptide/protein binds to at least one epitope in the alpha-chain of an HLA-DR molecule.

According to a further preferred embodiment, the peptide/protein binds to at least one epitope of the first domain of the alpha-chain of HLA-DR.

In a further preferred embodiment the peptide/protein binds to at least one epitope within the alpha-helix ranging from Glu⁵⁵ to Tyr⁷⁹ of the alpha-chain of HLA-DR.

In a further preferred embodiment the peptide/protein binds to at least one epitope in the beta-chain of an HLA-DR molecule.

According to a further preferred embodiment, the peptide/protein binds to at least one epitope of the first domain of the beta-chain of HLA-DR.

In a further preferred embodiment the IgG-form of said antigen-binding domain binds to said antigen with an affinity of less than 50 nM.

In a further preferred embodiment the IgG-form of said antigen-binding domain binds to said antigen with an affinity of less than 10 nM.

In a further preferred embodiment the antigen-binding domain comprises a monovalent antibody fragment selected from Fv, scFv, dsFv and Fab fragment.

In a further preferred embodiment the peptide/protein comprises an F(ab)'₂ antibody fragment or a mini-antibody fragment.

In a further preferred embodiment the peptide/protein is a multivalent composition comprising at least one full antibody selected from IgG1, IgG2a, IgG2b, IgG3, IgG4, IgA and IgM.

In a preferred embodiment the peptide/protein causes or leads to selective immunomodulation of the immune response that causes or leads to immunosuppression.

In a further preferred embodiment the immunosuppression is brought about by down-regulation of expression of said MHC class II antigen on said cells.

In a further preferred embodiment the immunosuppression is brought about by inhibition of the interaction between said cells, wherein said interaction leads or causes an immune response.

In a further preferred embodiment the immunosuppression is brought about by a killing mechanism of the target cell.

A multivalent composition of at least one peptide/protein according to the invention is capable of leading to cell death of activated cells without requiring any further additional measures and with limited immunogenic side effects on the treated patient. Further, the multivalent composition comprising a peptide/protein according to the invention has the capability of binding to at least one epitope on the target antigen, however, several epitope binding sites might be combined in one molecule.

In a further preferred embodiment the peptide/protein is a multivalent composition comprising at least two monovalent antibody fragments selected from Fv, scFv, dsFv and Fab fragments, and further comprises a cross-linking moiety or moieties.

In a further preferred embodiment the peptide/protein affects killing affects at least 50%, preferably at least 80%, of activated cells compared to killing of less than 15%, preferably less than 10%, of non-activated cells.

In a further preferred embodiment the peptide/protein induces a killing mechanism of the target cell that involves an innate pre-programmed process of said cell.

In a further preferred embodiment the peptide/protein induces a killing mechanism, which is not an apoptotic cell death process.

In a further preferred embodiment the peptide/protein induces a killing mechanism, which makes use of proteases other than caspases.

In a further preferred embodiment the peptide/protein comprises an antigen-binding domain which consists of a combination of a VH domain and a VL domain, wherein said combination is found in one of the clones selected from the list of MS-GPC1 to 8, MS-GPC10 or 11, MS-GPC14 to 16, MS-GPC8-6, or MS-GPC8-17 as shown in Table 2.

In a further preferred embodiment the peptide/protein comprises an antigen-binding domain, which consists of a combination of HuCAL VH2 and HuCAL Vλ1, wherein the VH CDR3 sequence is taken from the consensus CDR3 sequence
nnnnRGnFDn
wherein each n independently represents any amino acid residue; and
wherein the VL CDR3 sequence is taken from the consensus CDR3 sequence
   QSYDnnnn
wherein each n independently represents any amino acid residue.

The present invention also relates to a nucleic acid which encodes a peptide/protein according to the invention.

The invention further relates to variants of this nucleic acid which hybridise under stringent conditions with a nucleic acid as above, wherein said variants encode a peptide/protein according to the invention.

The invention further relates to a vector comprising at least one nucleic acid and/or at least one variant thereof according to the invention.

The invention further relates to a host cell harbouring nucleic acids, variants and/or vectors as described above.

According to the invention, the peptide/protein is prepared by a method comprising the step of expressing at least one nucleic acid and/or at least one variant according to the invention in a suitable expression system, which can be a cell free expression system, but is preferably a cell containing expression system, and isolating the protein therefrom. The nucleic acid for preparing the proteins according to the invention can be obtained by standard laboratory procedures well known to one of ordinary skill in the art (see, e.g. Ausubel et al., 1998)

The present invention further relates to a pharmaceutical composition containing at least one antigen-binding peptide/protein according to the invention, optionally together with a pharmaceutical acceptable carrier and/or diluent.

The peptide/protein according to the invention is preferably used for preparing a pharmaceutical composition for treating animals, preferably humans.

The peptide/protein according to the invention is preferably useful for the treatment or prevention of a condition characterised by MHC class II-mediated activation of T and/or B cells.

In a further preferred embodiment said treatment is the treatment or prevention of a condition characterised by expression of MHC class II expression at pathological sites of inflammation.

In a further preferred embodiment said treatment is the treatment or prevention of diseases of the immune system.

In a preferred embodiment the protein/peptide according to the invention is used in the treatment of diseases of the immune system including conditions such as rheumatoid arthritis, juvenile arthritis, multiple sclerosis, Grave's disease, narcolepsy, psoriasis, systemic lupus erythematosus, transplant rejection, graft vs. host disease, Hashimoto's disease, myasthenia gravis, pemphigus vulgaris, glomerulonephritis, thyroiditis, insulitis, primary biliary cirrhosis, irritable bowel disease and Sjogren syndrome.

The invention further relates to a diagnostic composition containing at least one peptide/protein and/or nucleic acid according to the invention optionally together with further reagents, such as buffers, for performing the diagnosis.

The present invention further relates to a method for immunosuppression comprising the step of contacting a lymphoid cell or a non-lymphoid cell which expresses MHC class II molecules with at least one peptide/protein according to the invention.

In an alternate preferred embodiment said immunosuppression is brought about by down-regulation of expression of said MHC class II antigen on said cells.

In an alternate embodiment said immunosuppression is brought about by inhibition of the interaction between said cells, wherein said interaction leads or causes an immune response.

In a further alternate embodiment said immunosuppression is brought about by killing of said cells.

Additionally, the present invention relates to a kit comprising (i) a peptide/protein according to the present invention, (ii) a detectable moiety or moieties, and (iii) reagents and/or solutions to effect and/or detect binding of (i) to an antigen.

### Figure Captions

### Figure 1

Specificity of the anti-HLA DR antibody fragments MS-GPC1 to 8, MS-GPC10 and 11 and MS-GPC14 to 16, isolated from the HuCAL library to HLA-DR protein, a mouse-human chimeric HLA protein and negative control proteins, lysozyme, transferrin, BSA and human γ-globulin. Specificity was assessed using standard ELISA procedures. A non-related antibody fragment (irr. scFv) was used as control.

### Figure 2

Reactivity of the anti-HLA-DR antibody fragments (MS-GPC1 to 8, MS-GPC10 and 11 and MS-GPC15 and 16) to various cells lines expressing MHC class II molecules. "+" represents strong reactivity as detected using standard immunofluorescence procedure. "+/-" represents weak reactivity and "-" represents no detected reactivity between a anti-HLA-DR antibody fragment and a particular cell line. "nt" represent reactivity which was not tested.

### Figure 3

Viability of activated cells to monovalent and cross-linked anti-HLA DR antibody fragments as accessed by trypan blue staining. Viability of GRANTA-519 cells was assessed 4 h after incubation with anti-HLA DR antibody fragments (MS-GPC-1, 6, 8 and 10) with and without anti-FLAG M2 mAb as cross-linking agent.

### Figure 4

Cytotoxicity on activated versus non-activated cells. MHH-PREB-1 cells are activated with Lipopolysaccharide and Interferon-gamma, and subsequently incubated for 4 h with 200 nM of anti-HLA DR antibody fragment MS-GPC-8 cross-linked using 100 nM of anti-FLAG M2 mAb. Minimal killing of control non-activated MHH-PREB-1 cells is seen.

### Figure 5

a. Incubation of Priess cells with the anti-HLA DR antibody fragment MS-GPC-8, cross-linked using the anti-FLAG M2 mAb, shows more rapid killing than a culture of Priess cells induced into apoptosis using anti-CD95 mAb. An Annexin V/PI staining technique identifies dead cells.
b. Incubation of Priess cells with the anti-HLA DR antibody fragment MS-GPC-8, cross-linked using the anti-FLAG M2 mAb, shows little evidence of an apoptotic mechanism compared to an apoptotic culture of Priess cells induced using anti-CD95 mAb. An Annexin V/PI staining technique identifies apoptotic cells.

### Figure 6

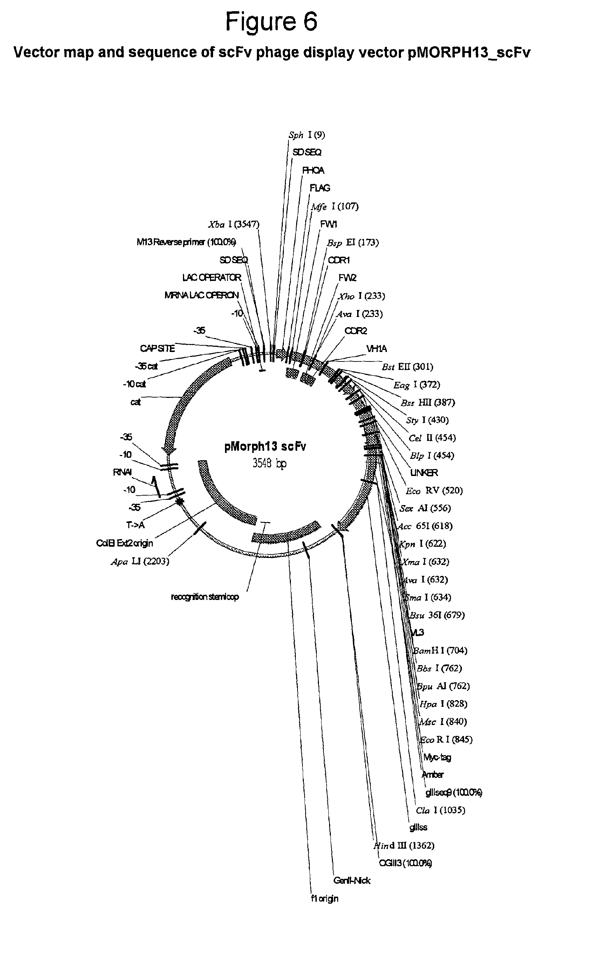

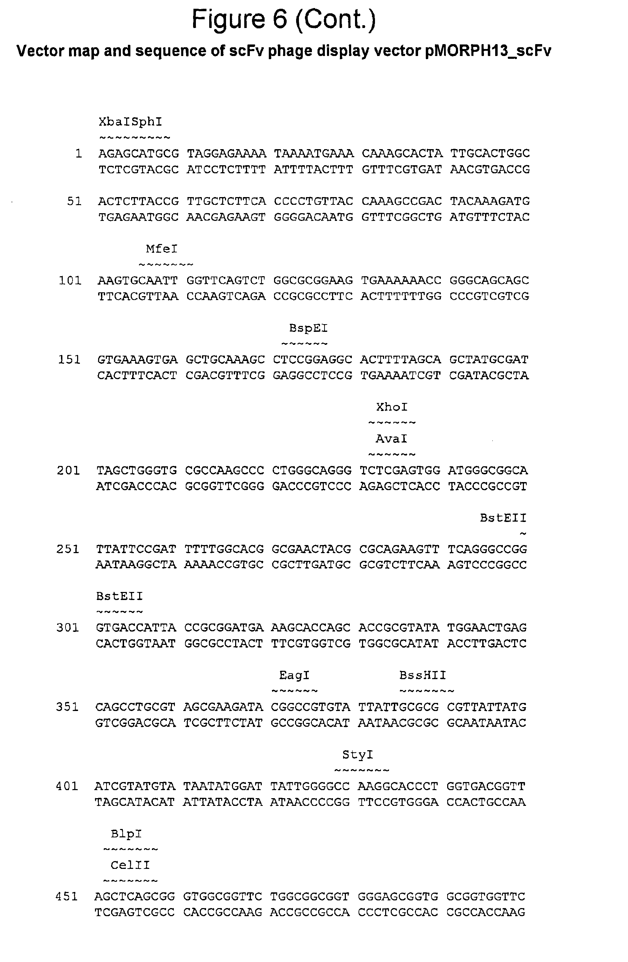

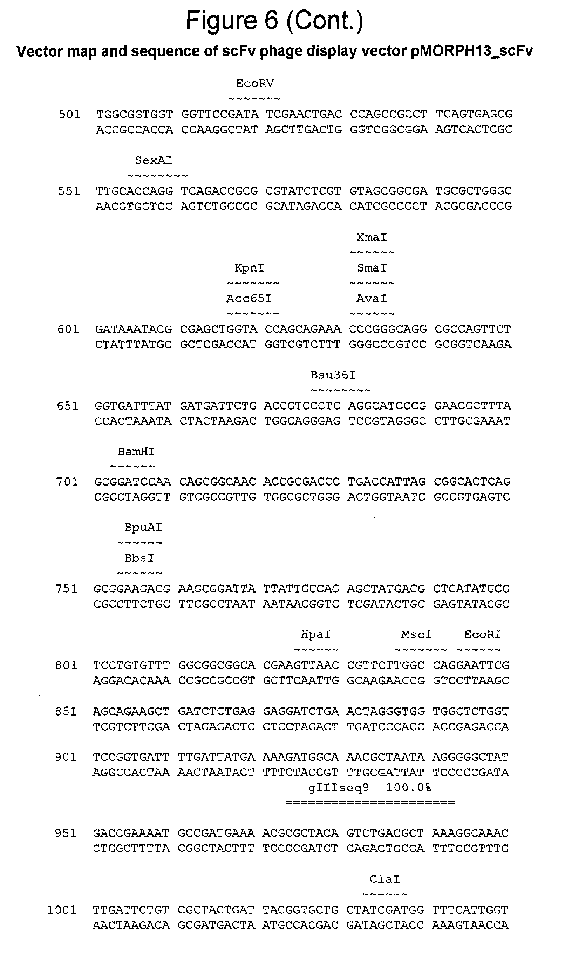

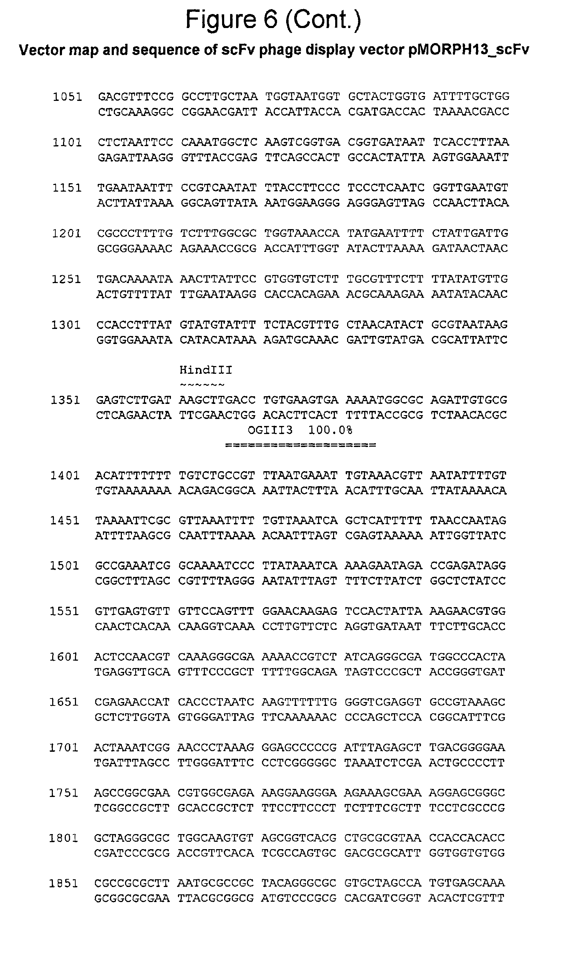

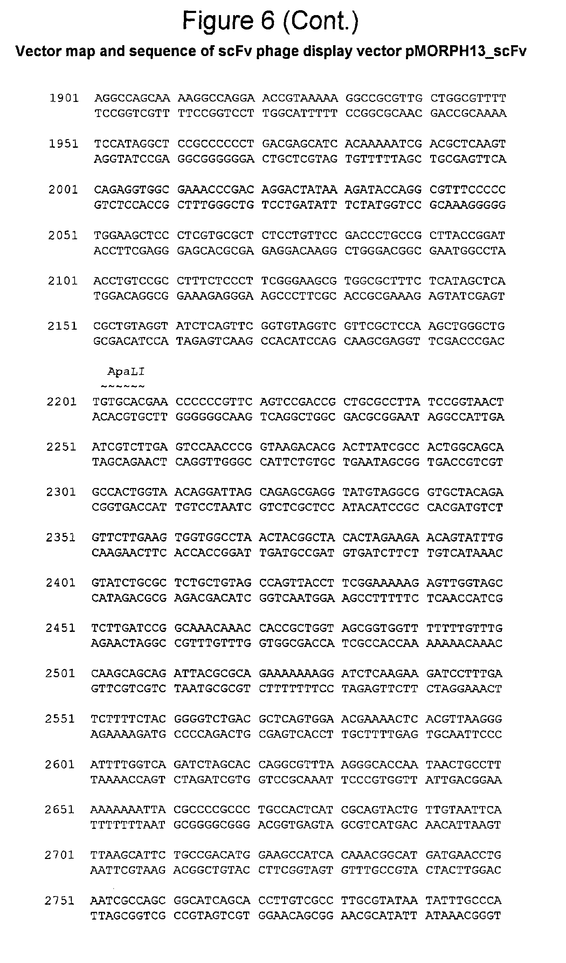

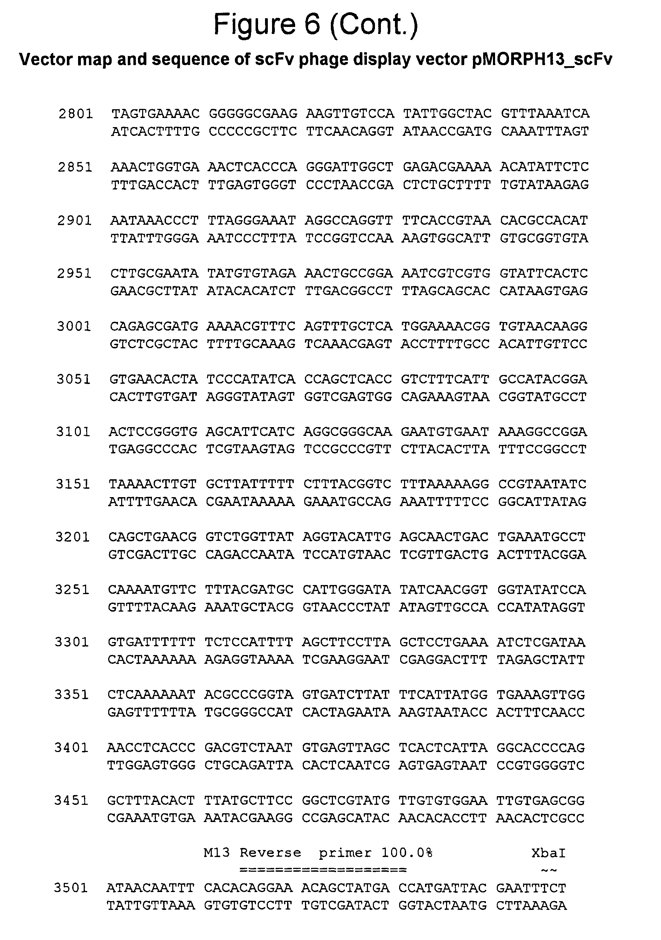

Vector map and sequence of scFv phage display vector pMORPH13_scFv. The vector pMORPH13_scFv is a phagemid vector comprising a gene encoding fusion between the C-terminal domain of the gene III protein of filamentous phage and a HuCAL scFv. In Fig. 6, a vector comprising a model scFv gene (combination of VH1A and Vλ3 (Knappik et al., 2000) is shown.

### Figure 7

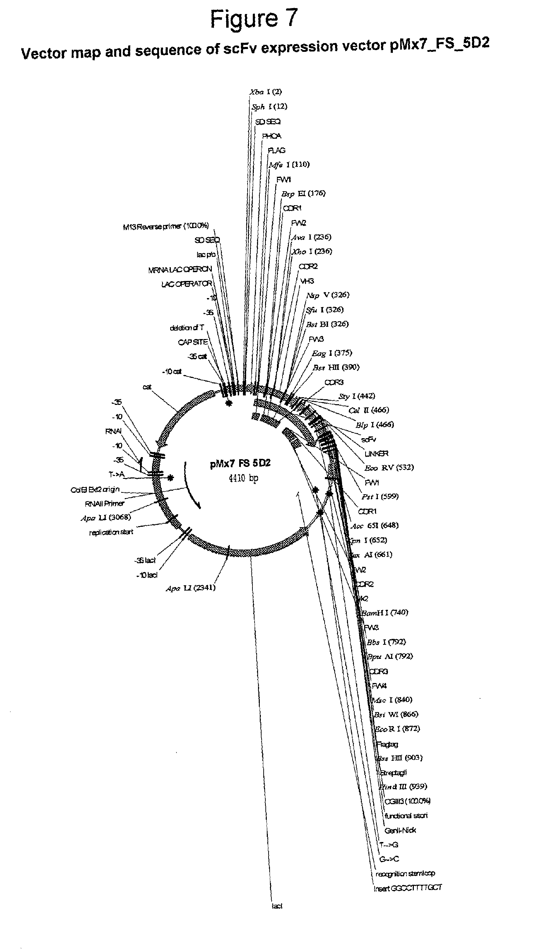

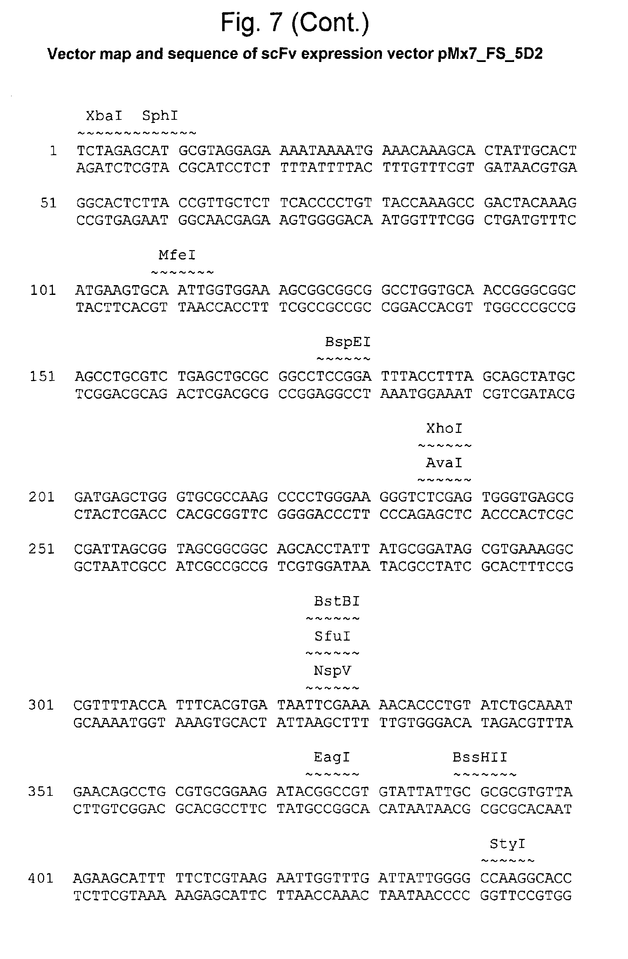

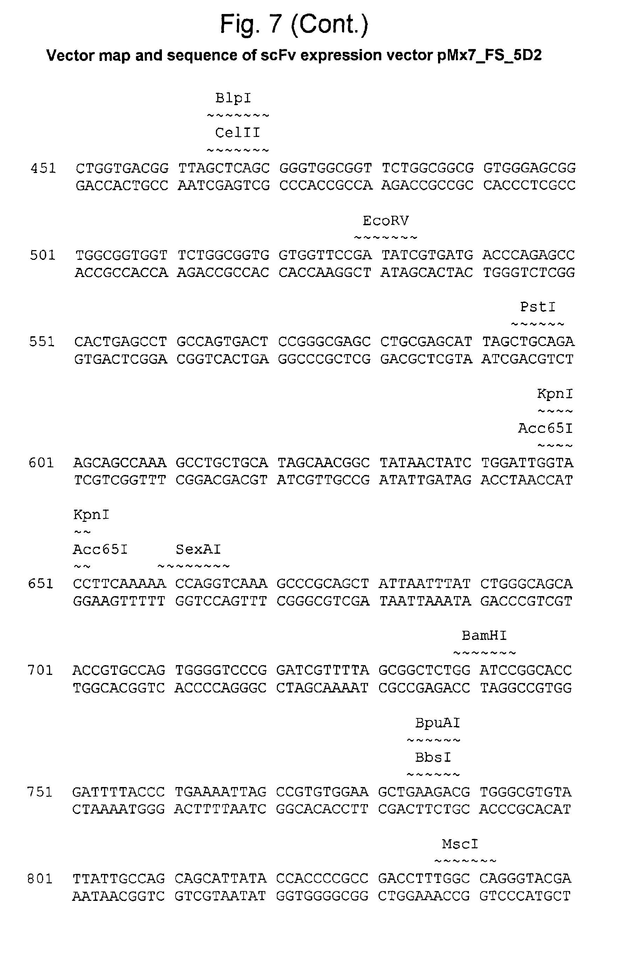

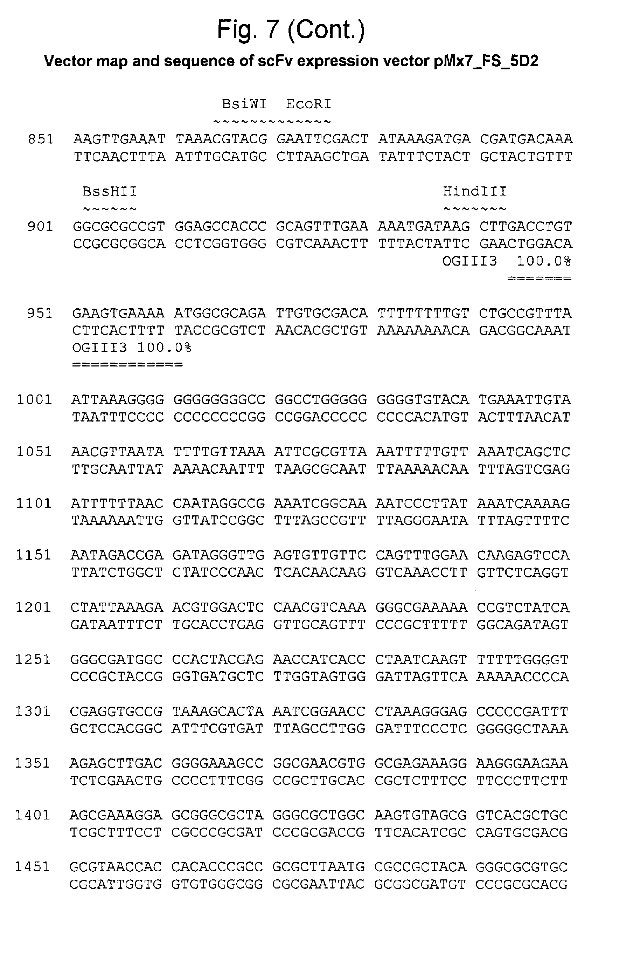

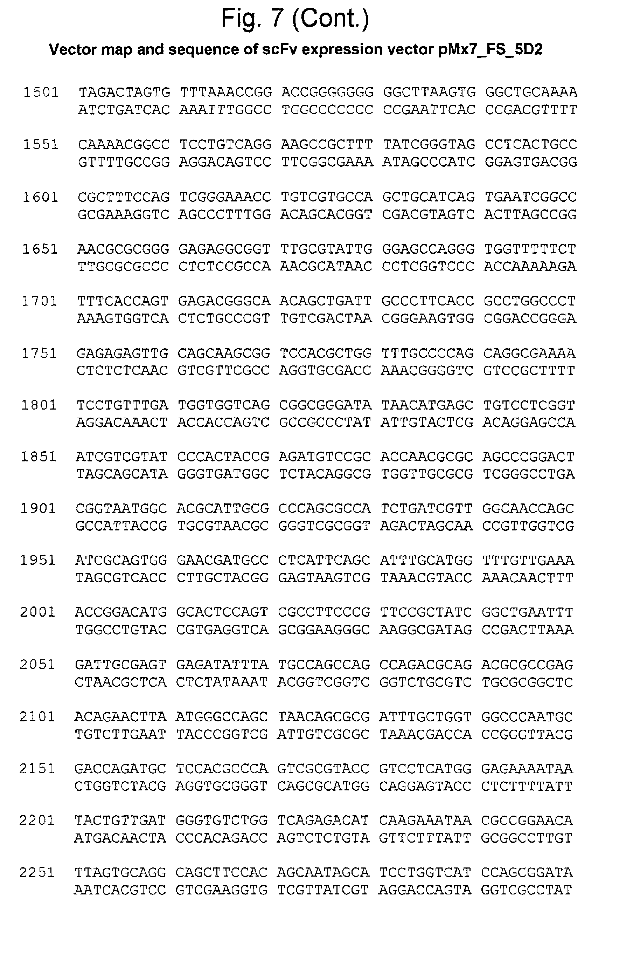

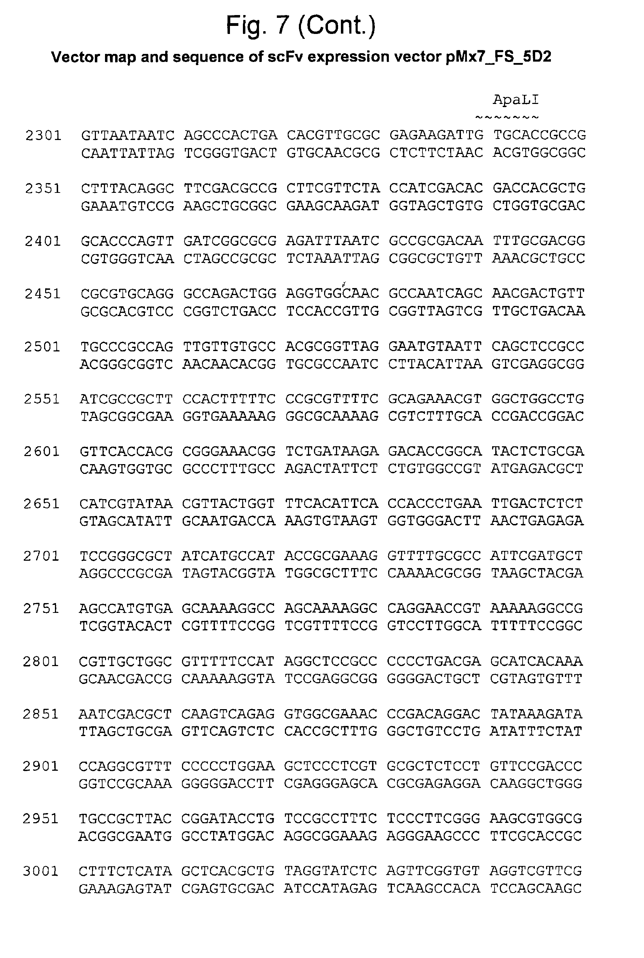

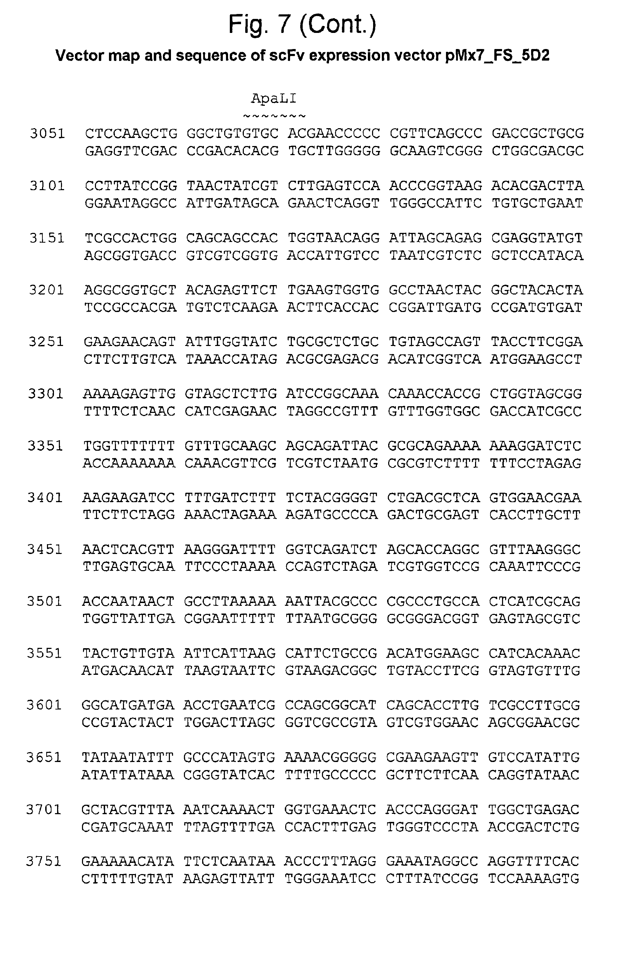

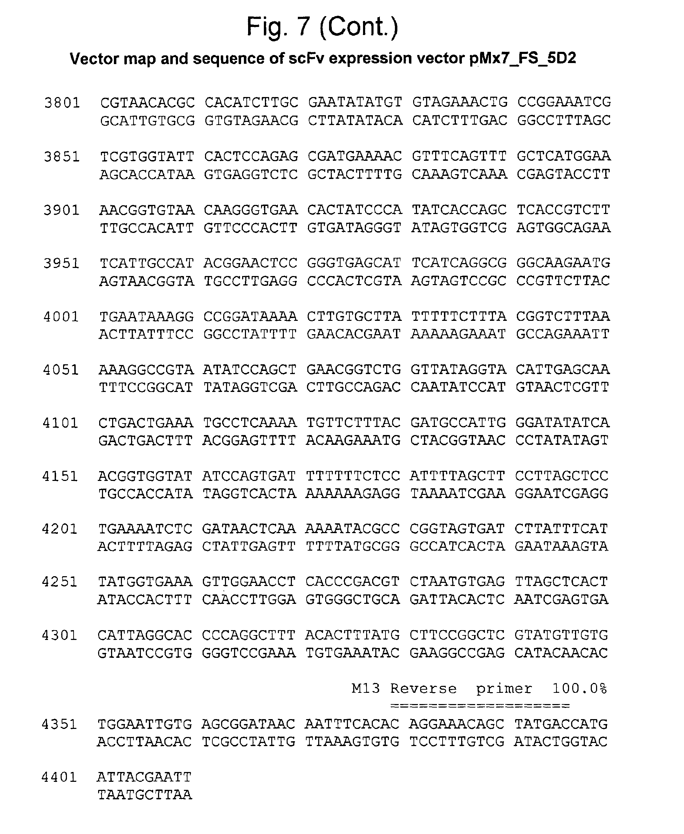

Vector map and sequence of scFv expression vector pMx7_FS_5D2. The expression vector pMx7_FS_5D2 leads to the expression of HuCAL scFv fragments (in Fig. 7, the vector comprises a gene encoding a "dummy" antibody fragment called "5D2") fused to a combination of a FLAG tag (Hopp et al., 1988; Knappik and Plückthun, 1994) and a STREP tag II (WSHPQFEK) (IBA GmbH, Göttingen, Germany; see: htttp://www.iba-go.de and Schmidt and Skerra, 1993; Schmidt and Skerra, 1994; Schmidt et al., 1996; Voss and Skerra, 1997).

### Figure 8

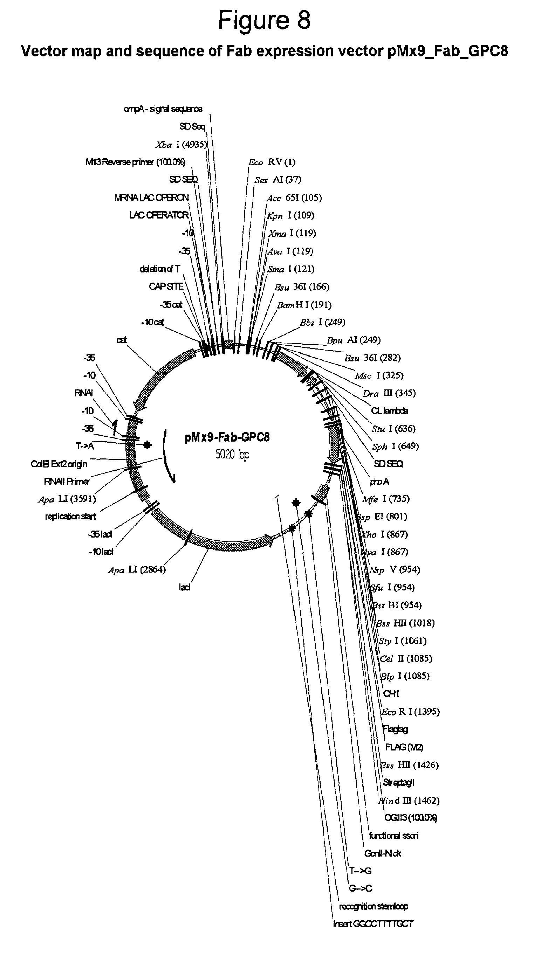

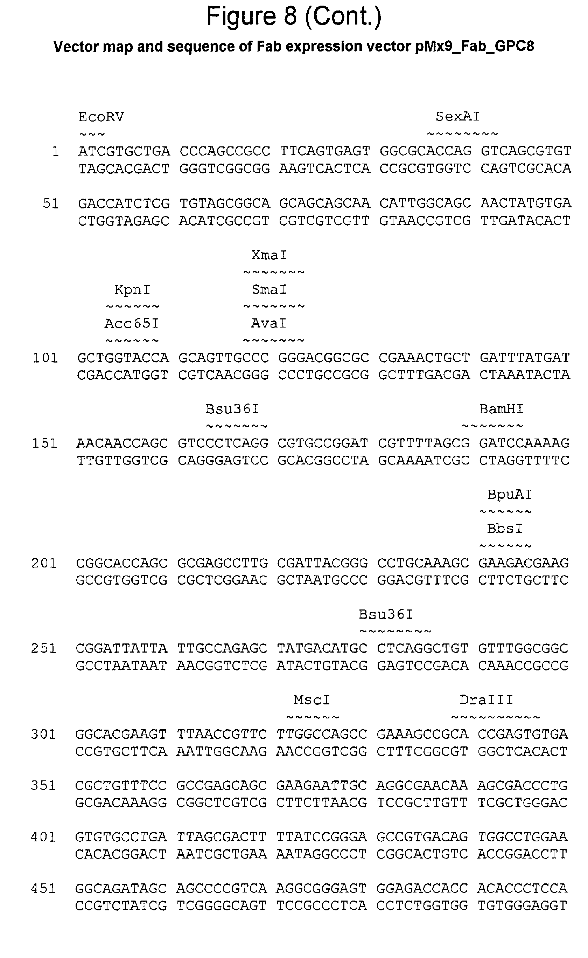

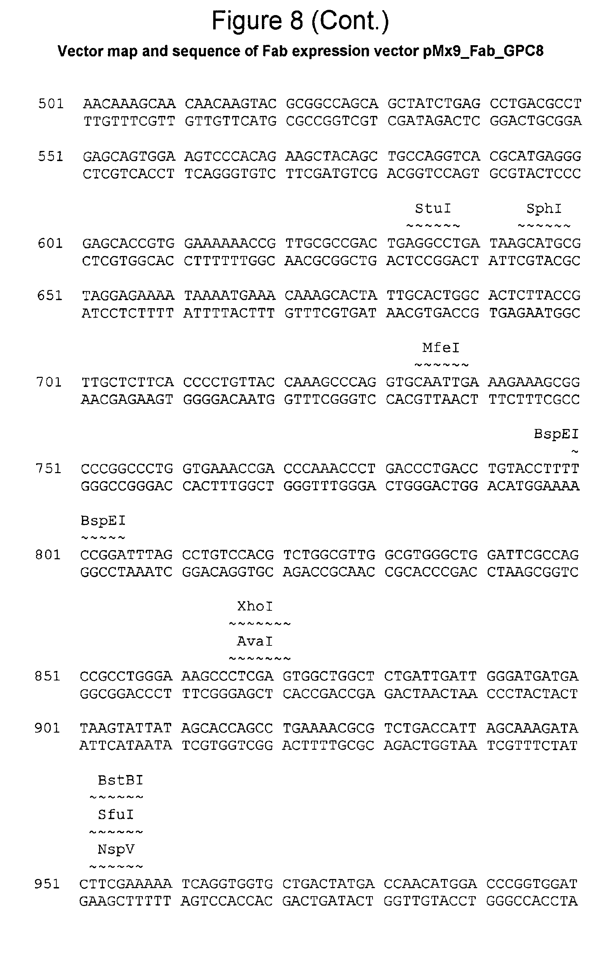

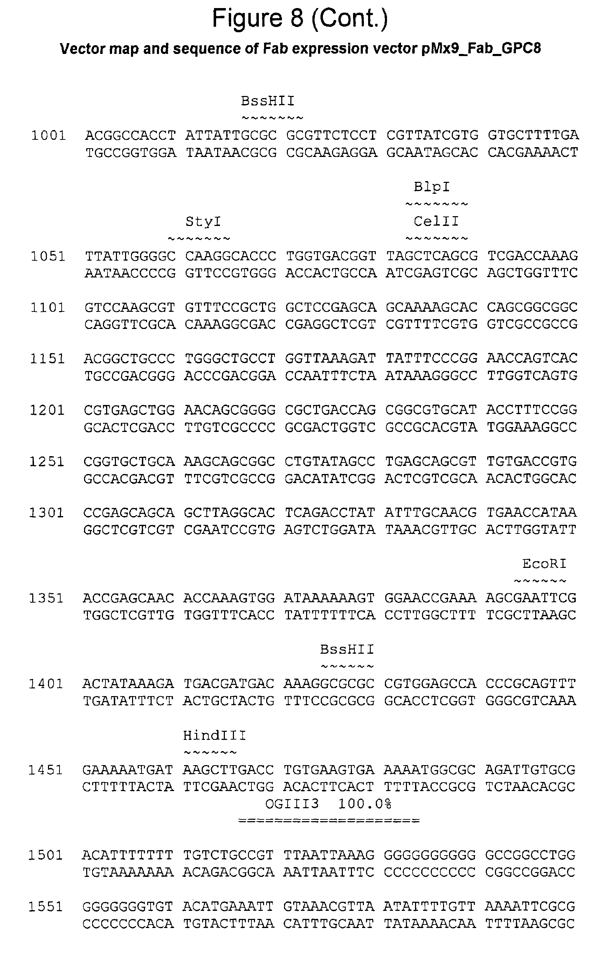

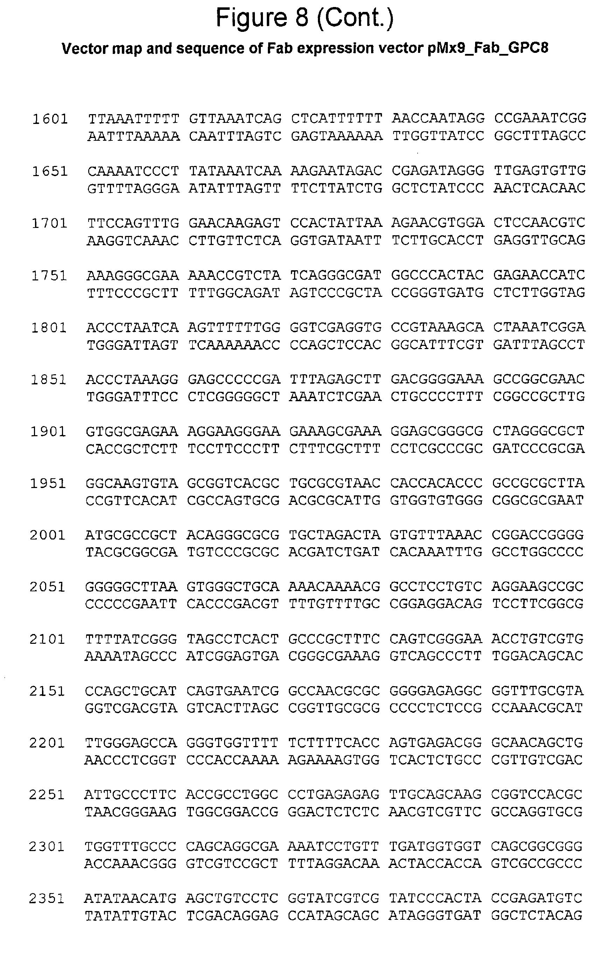

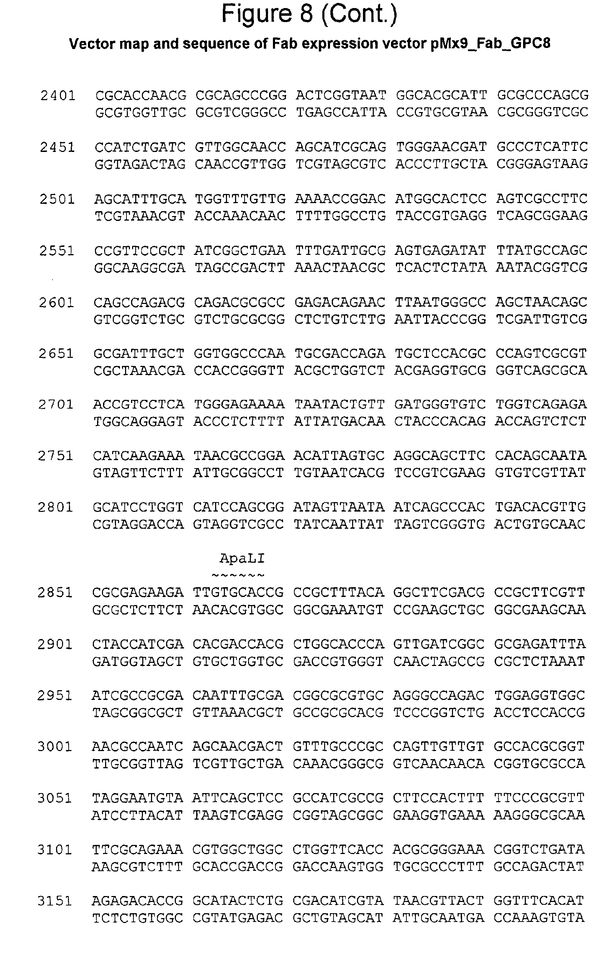

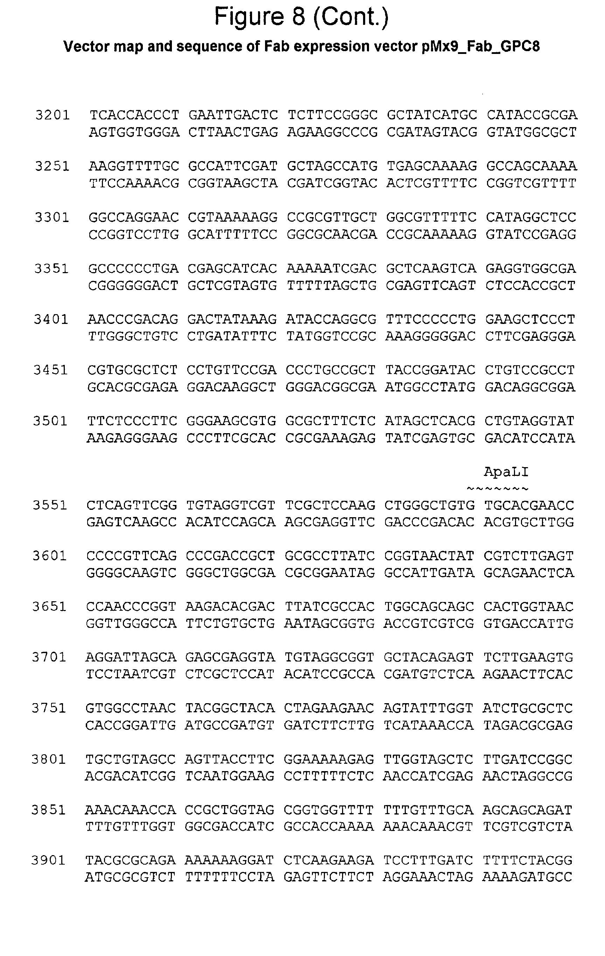

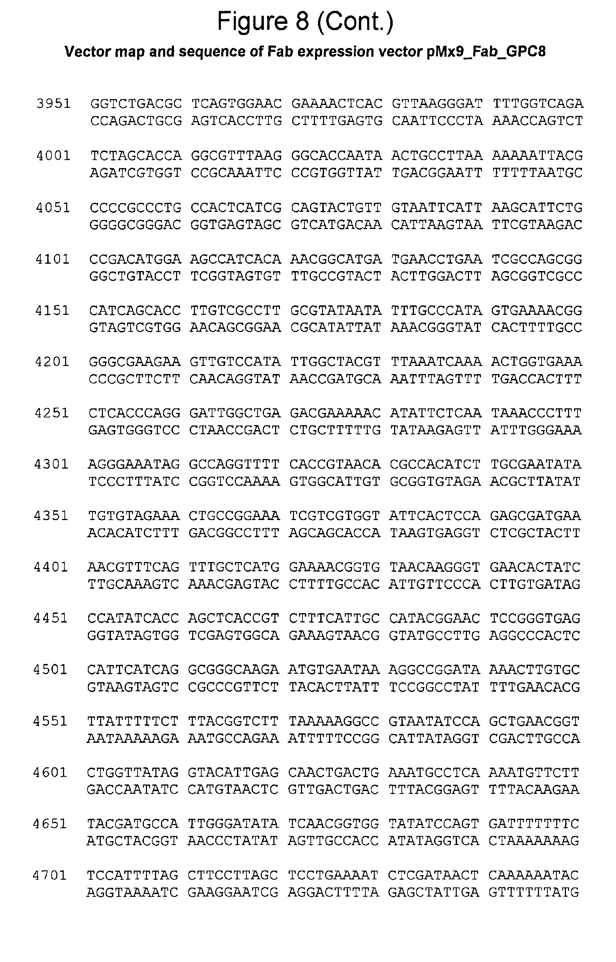

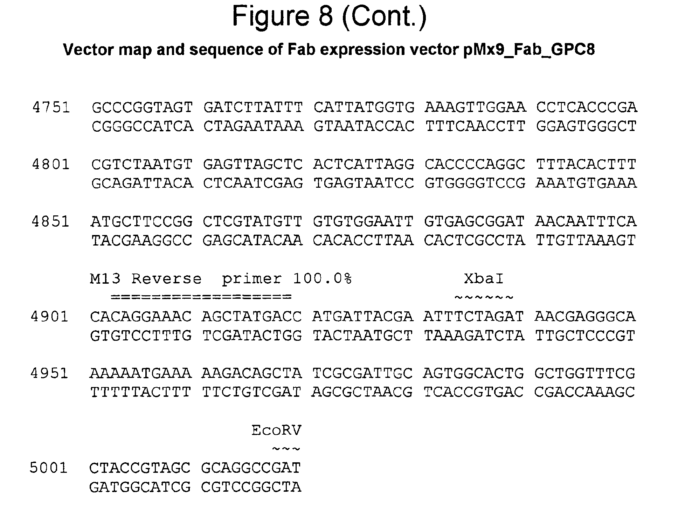

Vector map and sequence of Fab expression vector pMx9_Fab_GPC8. The expression vector pMx9_Fab_GPC8 leads to the expression of HuCAL Fab fragments (in Fig. 8, the vector comprises the Fab fragment MS-GPC8) fused to a combination of a FLAG tag (Hopp et al., 1988; Knappik and Plückthun, 1994) and a STREP tag II (WSHPQFEK) (IBA GmbH, Göttingen, Germany; see: htttp://www.iba-go.de and Schmidt and Skerra, 1993; Schmidt and Skerra, 1994; Schmidt et al., 1996; Voss and Skerra, 1997).

### Figure 9

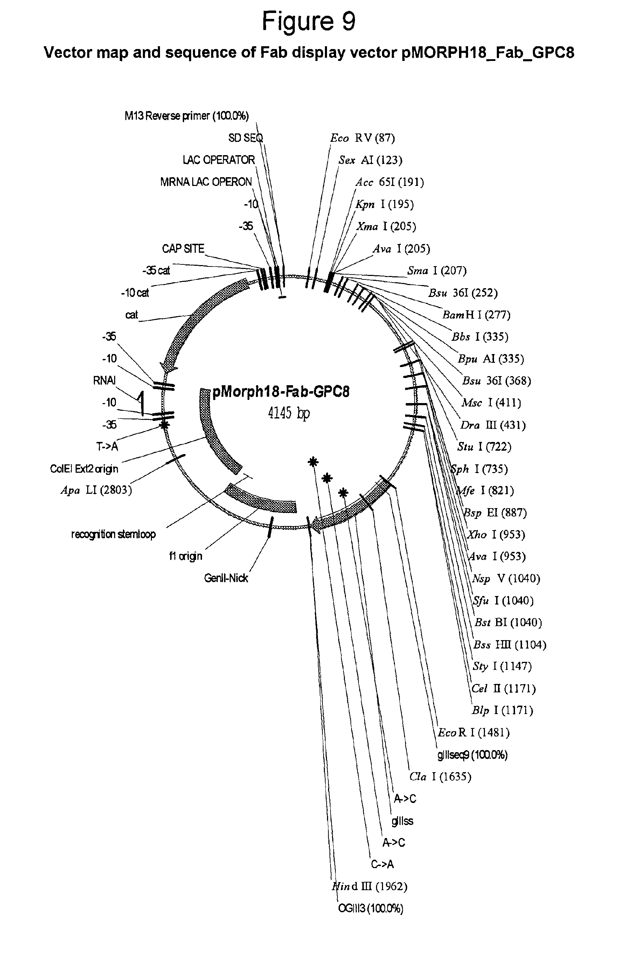

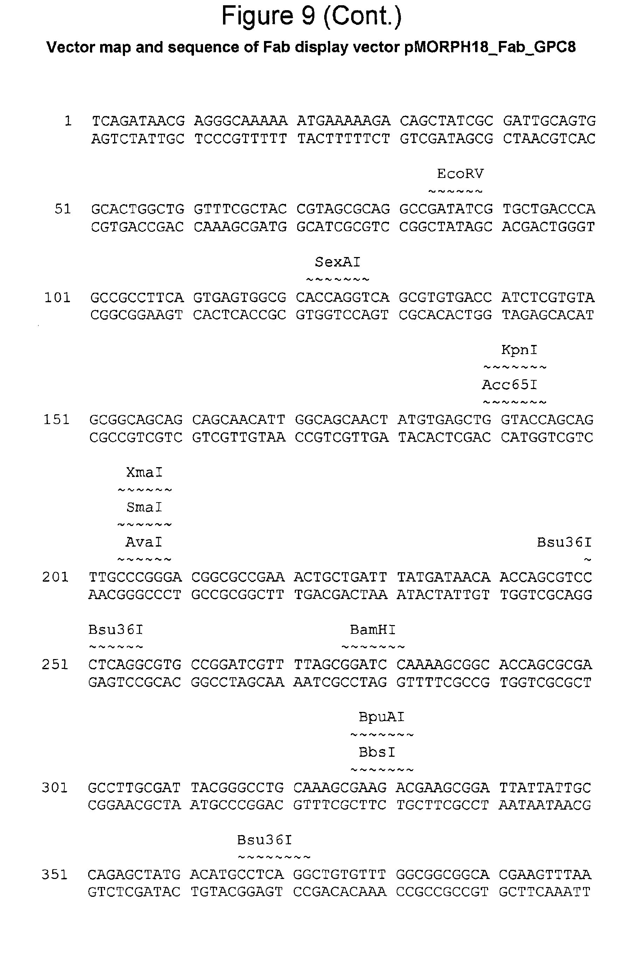

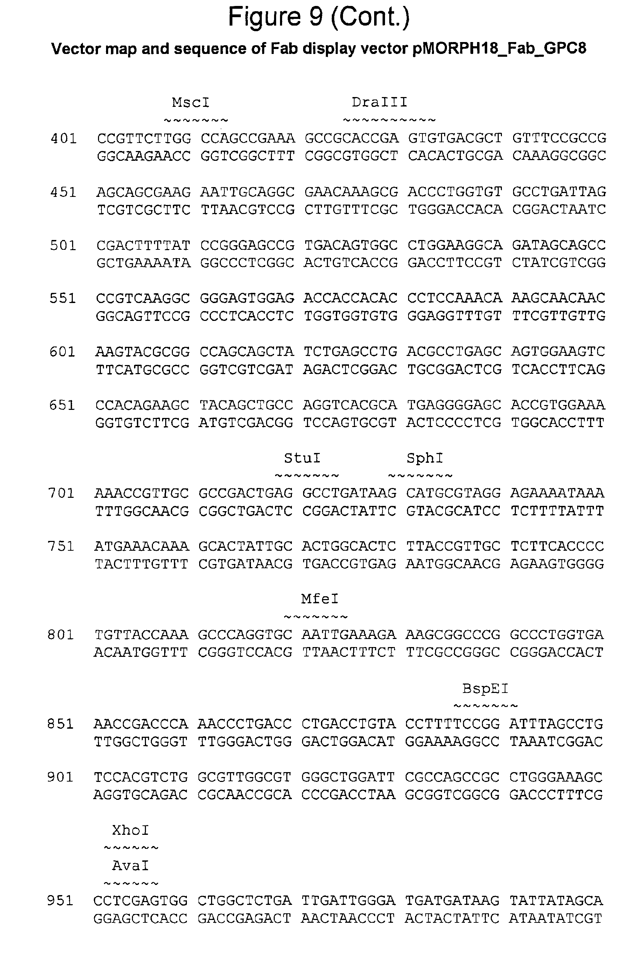

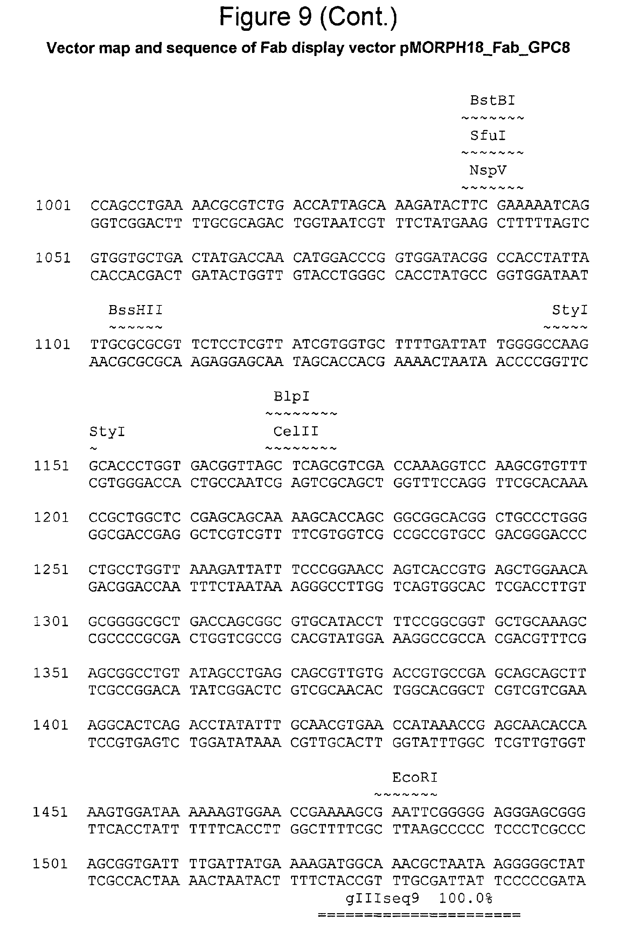

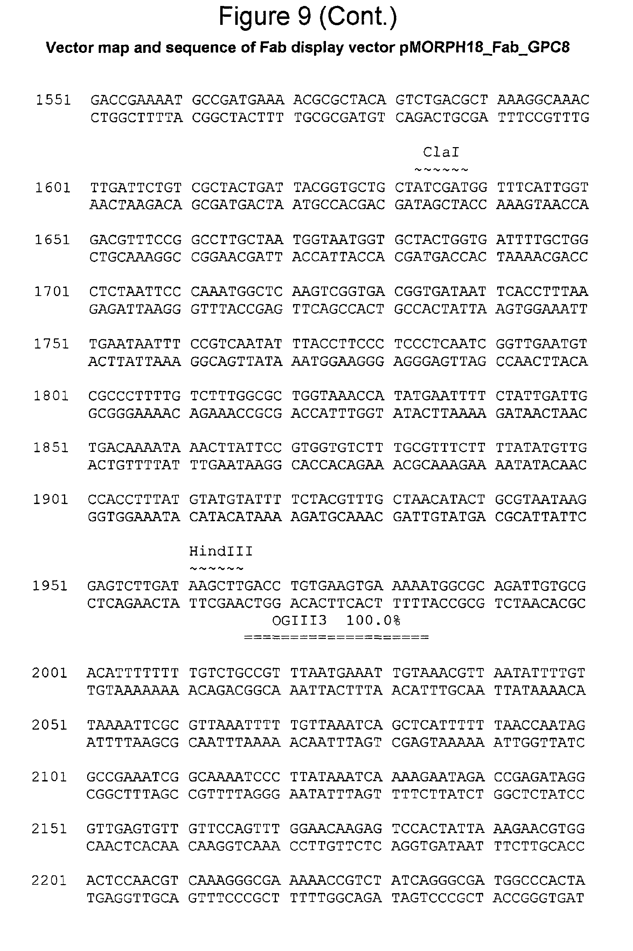

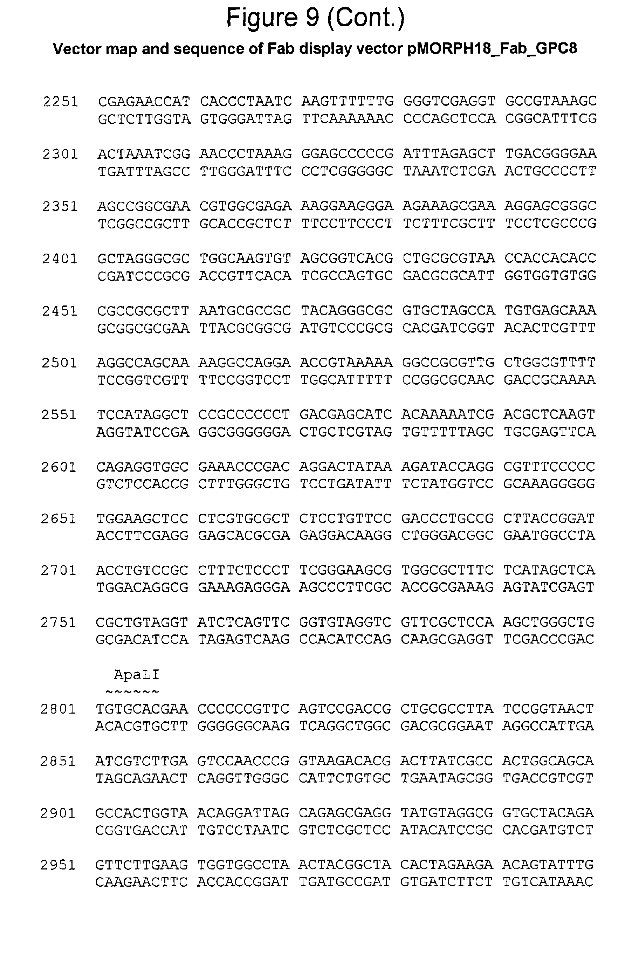

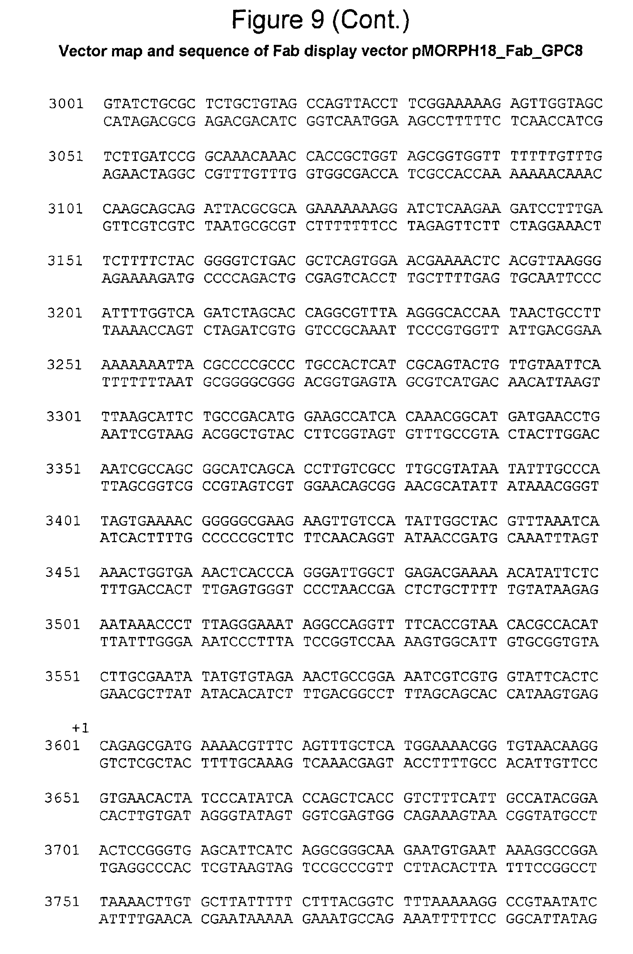

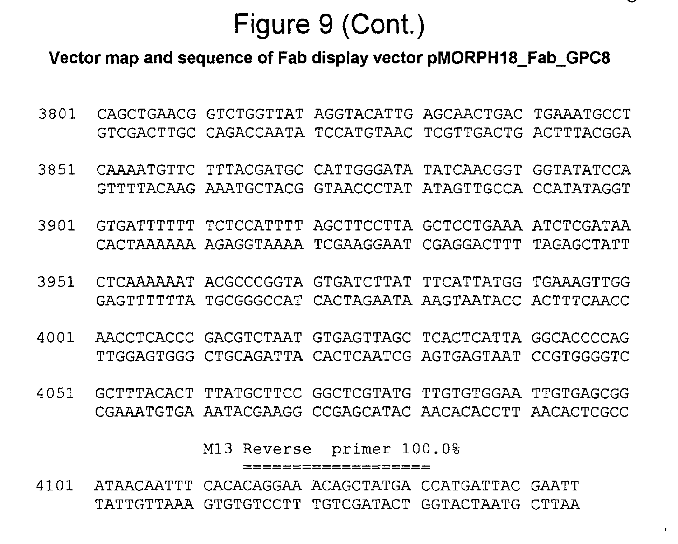

Vector map and sequence of Fab phage display vector pMORPH18_Fab_GPC8. The derivatives of vector pMORPH18 are phagemid vectors comprising a gene encoding fusion between the C-terminal domain of the gene III protein of filamentous phage and the VH-CH1 chain of a HuCAL antibody. Additionally, the vector comprises the separately encoded VL-CL chain. In Fig. 9, a vector comprising the Fab fragment MS-GPC8 is shown.

### Figure 10

Amino acid sequences of VH and VL domains of MS-GPC1 to 8, MS-GPC10 and 11, MS-GPC14 to 16, MS-GPC8-6, and MS-GPC8-17.

The following examples illustrate the invention.

### Examples

(All buffers, solutions or procedures without explicit reference can be found in standard textbooks, for example Current Protocols of Immunology (1997 and 1999) or Sambrook et al., 1989.)

### A. Preparation of a human antigen

To demonstrate that we could identify immunomodulatory antigen-binding domains of human composition, we prepared a purified form of a human antigen, the human MHC class II DR protein (DRA*0101/DRB1*0401) from PRIESS cells (Gorga et al., 1984; Gorga et al., 1986; Gorga et al., 1987; Stern et al., 1992) as follows.

First, PRIESS cells (ECACC, Salisbury UK) were cultured in RPMI and 10% FCS using standard conditions, and 10¹⁰ cells were lysed in 200 ml PBS (pH 7.5) containing 1% NP-40, 25 mM iodoacetamide, 1 mM PMSF and 10 mg/l each of the protease inhibitors chymostatin, antipain, pepstatin A, soybean trypsin inhibitor and leupeptin. The lysate was centrifuged at 10.000 g (30 minutes, 4°C) and the resulting supernatant was supplemented with 40 ml of an aqueous solution containing 5 % sodium deoxycholate, 5 mM iodoacetamide and 10 mg/l each of the above protease inhibitors and centrifuged at 100.000 g for two hours (4°C). To remove material that bound non-specifically and endogenous antibodies, the resulting supernatant was made 0.2 mM with PMSF and passed overnight (4°C) through a rabbit serum affigel-10 column (5 ml) followed by a Protein G Sepharose Fast Flow column (2 ml; Pharmacia) using a flow rate of 0.2 ml/min.

Second, the pre-treated lysate was batch incubated with 5 ml LB3.1-Protein G Sepharose Fast Flow beads (Stem et al., 1993) overnight at 4°C using gentle mixing, and then transferred into a small column which was then washed extensively with three solutions: (1) 100 ml of a solution consisting of 50 mM Tris/HCl (pH 8.0), 150 mM NaCI, 0.5 % NP-40, 0.5 % sodium deoxycholate, 10 % glycerol and 0.03 % sodium azide at a flow rate of 0.6 ml/min); (2) 25 ml of a solution consisting 50 mM Tris/HCl (pH 9.0), 0.5 M NaCI, 0.5 % NP-40, 0.5 % sodium deoxycholate, 10 % glycerol and 0.03 % sodium azide at a flow rate of 0.9 ml/min; (3) 25 ml of a solution consisting of 2 mM Tris/HCl (pH 8.0), 1 % octyl-β-D-glucopyranoside, 10 % glycerol and 0.03 % sodium azide at a flow rate of 0.9 ml/min.

Third, MHC-class II DR protein (DRA*0101/DRB1*0401) was eluted using 15 ml of a solution consisting of 50 mM diethylamine/HCl (pH 11.5), 150 mM NaCI, 1 mM EDTA, 1 mM EGTA, 1 % octyl-β-D-glucopyranoside, 10 % glycerol, 10 mM iodoacetamide and 0.03 % sodium azide at a flow rate of 0.4 ml/min. 800 µl fractions were immediately neutralised with 100 µl 1M Tris/HCl (pH 6.8), 150 mM NaCI and 1 % octyl-β-D-glucopyranoside. The incubation of the lysate with LB3.1-Protein G Sepharose Fast Flow beads was repeated until the lysate was exhausted of MHC protein. Pure eluted fractions of the MHC class II DR protein (as analysed by SDS-PAGE) were pooled and concentrated to 1.0-1.3 g/l using Vivaspin concentrators with a 30 kDa molecular weight cut-off. Approximately 1 mg of the MHC-class II DR preparation was re-buffered with PBS containing 1 % octyl-β-D-glucopyranoside using the same Vivaspin concentrator to enable direct coupling of the protein to BIAcore CM5 chips.

### B. Screening of HUCAL

### B.1. Introduction

We identified antigen binding antibody fragments of human composition against the human antigen (DRA*0101/DRB1*0401) from a human antibody library based on a novel concept that has been recently developed (Knappik et al., 2000). A consensus framework resulting in a total of 49 different frameworks here represents each of the VH- and VL-subfamilies frequently used in human immune responses. These master genes were designed to take into account and eliminate unfavourable residues promoting protein aggregation as well as to create unique restriction sites leading to modular composition of the genes. In HuCAL-scFv, both the VH- and VL-CDR3 encoding regions of the 49 master genes were randomised.

### B.2. Phagemid rescue, phage amplification and purification

The HuCAL-scFv (Knappik et al., 2000) library, cloned into a phagemid-based phage display vector pMORPH13_scFv (see. 6), in *E.coli* TG-1 was amplified in 2 x TY medium containing 34 µg/ml chloramphenicol and 1 % glucose (2 x TY-CG). After helper phage infection (VCSM13) at 37°C at an OD₆₀₀ of about 0.5, centrifugation and resuspension in 2 x TY / 34 µg/ml chloramphenicol / 50 µg/ml kanamycin / 0.1 mM IPTG, cells were grown overnight at 30°C. Phage were PEG-precipitated from the supernatant (Ausubel et al., 1998), resuspended in PBS/20% glycerol and stored at -80°C. Phage amplification between two panning rounds was conducted as follows: mid-log phase TG1-cells were infected with eluted phage and plated onto LB-agar supplemented with 1% of glucose and 34 µg/ml of chloramphenicol. After overnight incubation at 30°C colonies were scraped off, adjusted to an OD₆₀₀ of 0.5 and helper phage added as described above.

### B.3. Manual solid phase panning

Wells of MaxiSorp™ microtiterplates (Nunc) were coated with MHC-class II DRA*0101/DRB1*0401 (prepared as above) dissolved in PBS (2 µg/well). After blocking with 5% non-fat dried milk in PBS, 1-5 x 10¹² HuCAL-scFv phage purified as above were added for 1h at 20°C. After several washing steps, bound phages were eluted by pH-elution with 100 mM triethylamine and subsequent neutralisation with 1M TRIS-CI pH 7.0. Three rounds of panning were performed with phage amplification conducted between each round as described above.

### B.4. Mixed solid phase/whole cell panning

Three rounds of panning and phage amplification were performed as described in B.3. and B.2. with the exception that in the second round between 1 x 10⁷ and 5 x 10⁷ PRIESS cells in 1 ml PBS/10% FCS were used in 10 ml Falcon tubes for whole cell panning. After incubation for 1h at 20°C with the phage preparation, the cell suspension was centrifuged (2000 rpm for 3 min) to remove non-binding phage, the cells were washed three times with 10 ml PBS, each time followed by centrifugation as described. Phage that specifically bound to the cells were eluted off by pH-elution using 100 mM HCI. Alternatively, binding phage could be amplified by directly adding E.coli to the suspension after triethlyamine treatment (100 mM) and subsequent neutralization.

### B.5 Identification of HLA-DR binding scFv fragments

Clones obtained after three rounds of solid phase panning (B.3) or mixed solid phase/whole cell panning (B.4) were screened by FACS analysis on PRIESS cells for binding to HLA-DR on the cell surface. For expression, the scFv fragments were cloned via Xbal/EcoRI into pMx7_FS as expression vector (see Fig. 7). Expression conditions are shown below in Example C.2.
Aliquots of 10⁶ Priess cells were transferred at 4°C into wells of a 96-well microtiterplate. ScFv in blocking buffer (PBS/5% FCS) were added for 60 min and detected with the using an anti-FLAG M2 antibody (Kodak) (1:5000 dilution) followed by a polyclonal goat anti-mouse IgG antibody-R-Phycoerythrin-conjugate (Jackson ImmunoResearch, 115-116-146, F(ab')₂ fragment) (1:200 dilution). Cells were fixed in 4% paraformaldehyde for storage at 4°C. 10⁴ events were collected for each assay on the FACS-Calibur (Becton Dickinson).

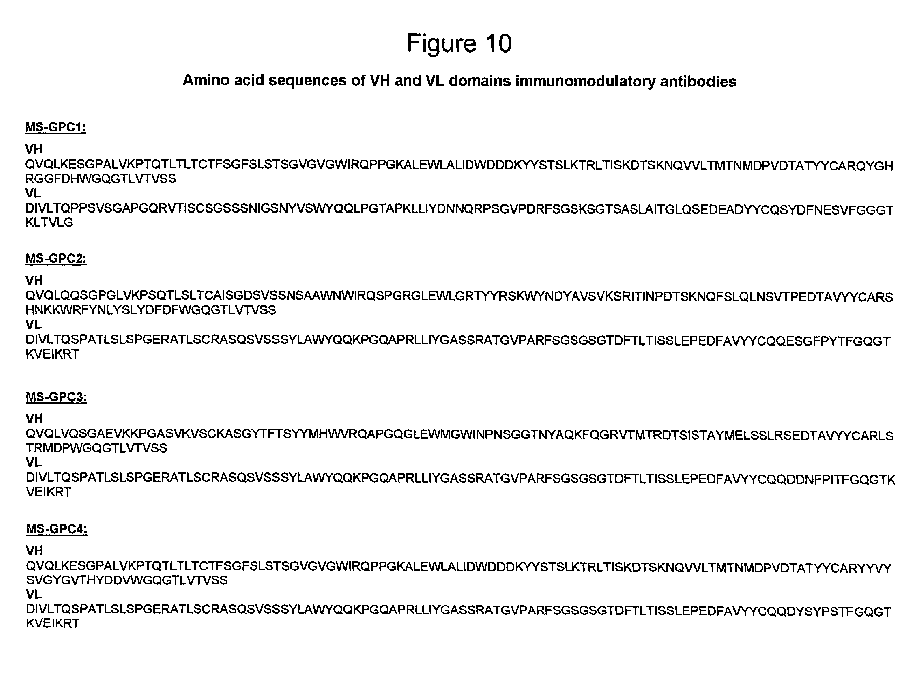

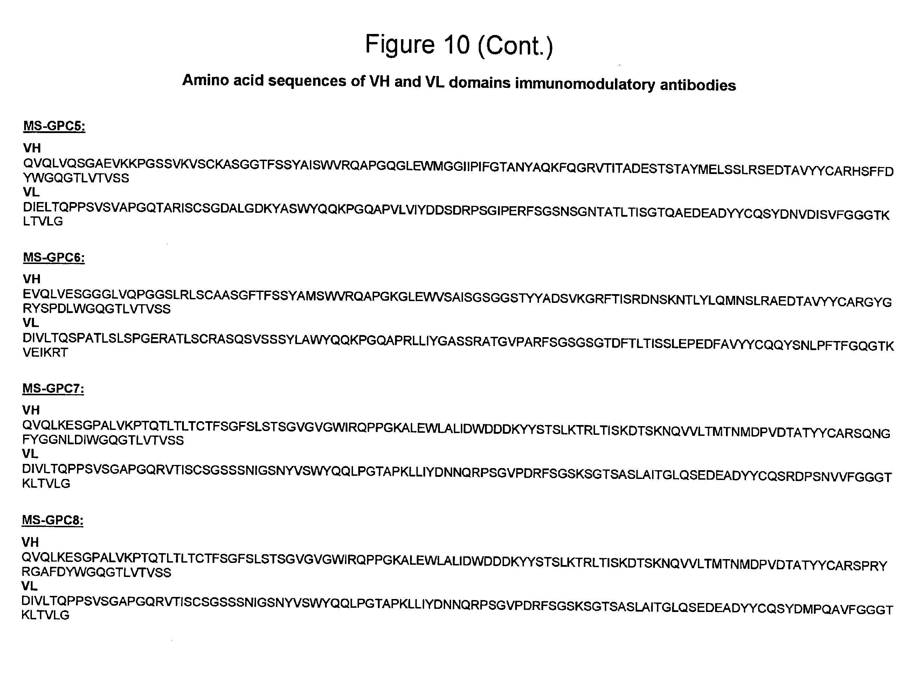

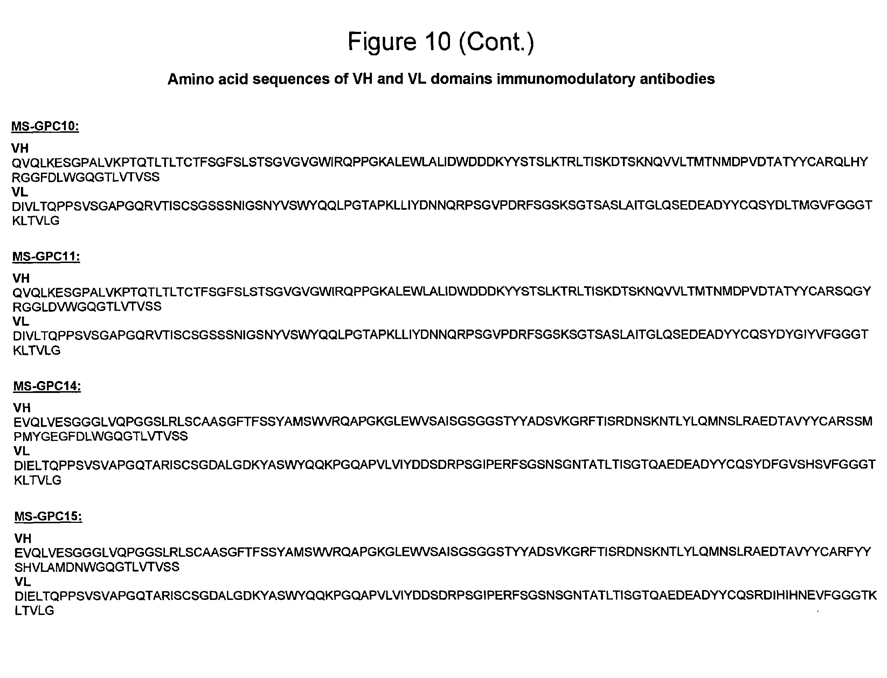

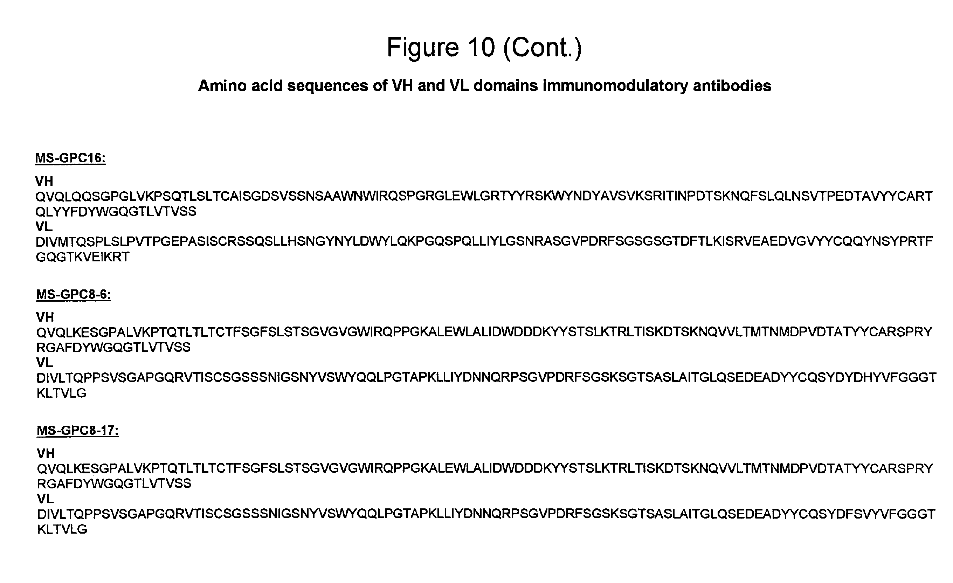

Only fifteen out of over 500 putative binders were identified which specifically bound to Priess cells. These clones are further analysed for their immunomodulatory activity as described below. Table 2 contains the sequence characteristics of clones MS-GPC1 to 8, MS-GPC10 and 11, MS-GPC14 to 16 identified thereby. The VH and VL families and the CDR3s listed refer to the HuCAL consensus-based antibody genes as described (Knappik et al., 2000) the full sequences of the VH and VL domains are shown in Figure 10.

### C. Generation of Fab-fragments

### C.1. Conversion of scFv to Fab

Both heavy and light chain variable domains of scFv fragments were cloned into pMx9_Fab (Fig. 8), the heavy chain variable domains as Mfel / Styl-fragments, the variable domains of the kappa light chains as EcoRV/ BsiWI-fragments. The lambda chains were first amplified from the corresponding pMORPH13_scFv vector as template with PCR-primers CRT5 (5' primer) and CRT6 (3' primer), wherein CRT6 introduces a unique DraIII restriction endonuclease site. 
CRT5: 5' GTGGTGGTTCCGATATC 3'
CRT6: 5' AGCGTCACACTCGGTGCGGCTTTCGGCTGGCCAAGAACGGGTTA 3'
 The PCR product is cut with EcoRV / DraIII and cloned into pMx9_Fab (see Fig. 8). The Fab light chains could be detected with a polyclonal goat anti-human IgG antibody-R-Phycoerythrin-conjugate (Jackson ImmunoResearch, 109-116-088, F(ab')₂ fragment) (1:200 dilution).

### C.2. Expression and purification of HuCAL-antibody fragments in E.coli

Expression in E.coli cells (JM83) of scFv and Fab fragments from pMx7_FS or pMx9_Fab, respectively, were carried out in one litre of 2 x TY-medium supplemented with 34 µg/ml chloramphenicol. After induction with 0.5 mM IPTG (scFv) or 0.1 mM IPTG (Fab), cells were grown at 22°C for 12 hours. Cell pellets were lysed in a French Press (Aminco) in 20 mM sodium phosphate, 0.5 M NaCI, and 10 mM imidazole (pH 7.4). Cell debris was removed by centrifugation and the clear supernatant filtered through 0.2 µm pores before subjecting it to STREP tag purification using a Streptactin matrix and purification conditions according to the supplier (IBA GmbH, Göttingen, Germany). Purification by size exclusion chromatography (SEC) was performed as described by Rheinnecker et al. (1996). The apparent molecular weights were determined by SEC with calibration standards and confirmed in some instances by coupled liquid chromatography-mass spectrometry (TopLab GmbH, Munich, Germany).

### D. HLA-DR specificity assay and epitope mapping

We conducted a binding-specificity test to demonstrate that antigen-binding domains selected from the HuCAL library bound specifically to the human MHC class II antigen. Using a standard ELISA procedure, scFv and Fab fragments selected from the HuCAL library were tested for reactivity with the following antigens: HLA-DR (DRA*0101/DRB1*0401), chimeric DR-IE (consisting of the N-terminal domains of DRA*0101 and DRB1*0401 with the remaining molecule derived from a murine class II homologue IEd. (Ito et al., 1996), and a set of negative control proteins comprising lysozyme, transferrin, BSA and human γ globulin.

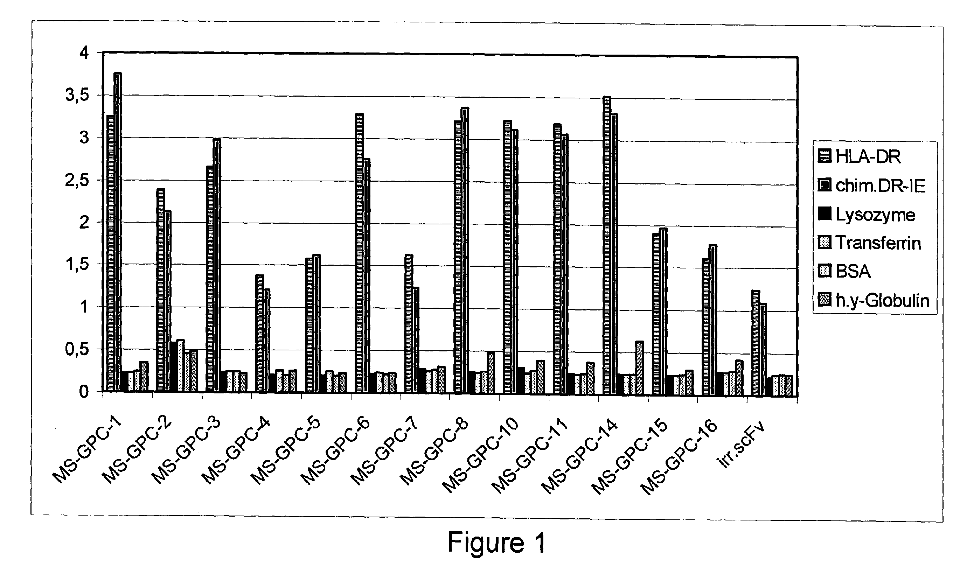

Figure 1 shows that 13 anti-HLA DR antibody fragments (MS-GPC1 to 8, MS-GPC10 and 11 and MS-GPC14 to 16) demonstrated better specificity for HLA-DR proteins than for control proteins. A non-related antibody fragment (Irr.scFv) showed very little specific reactivity to the HLA DR proteins. Indeed, any reactivity observed was shown to result from contaminating IgG within the preparation of MHC class II molecules.

We reasoned that the specific anti-HLA DR antibody fragments that were selected from the HuCAL library were recognising at least one epitope on the N-terminal extracellular protein of the human antigen. We conducted a series of experiments to confirm this hypothesis and to further define the epitope of binding for these anti-HLA DR antibody fragments.

First, 12 of the anti-HLA DR antibody fragments (MS-GPC1 to 8, MS-GPC10 and 11, MS-GPC15 and 16) were tested for reactivity against a panel of Epstein-Barr virus transformed B cell lines obtained from ECACC (Salisbury UK), each homozygous for one of the most frequent DR alleles in human populations, and to a series of L cells transfected to express human class II isotypes other than DRB1: L105.1, L257.6, L25.4, L256.12 & L21.3 that express the molecules DRB3*0101, DRB4*0101, DP0103/0402, DP 0202/0201, and DQ0201/0602 respectively (Klohe et al., 1998).

Reactivity of the antigen-binding fragment to the panel of cell-lines expressing various MHC-class II molecules was demonstrated using an immunofluorescence procedure as for example, described by Otten et al (1997). Staining was performed on 2x10⁵ cells using an anti-FLAG M2 antibody (Sigma) as the second reagent against the M2 tag carried by each anti-HLA DR antibody fragment and a fluorescein labelled goat anti-mouse Ig (Pharmingen) as a staining reagent. Cells were incubated at 4°C for 60 min with a predetermined dilution of the anti-HLA DR antibody fragment, followed by the second and third antibody at concentrations determined by the manufacturers. Cells were washed between incubation steps. Finally the cells were washed and subjected to analysis by a FACS Calibur (Becton-Dickinson).

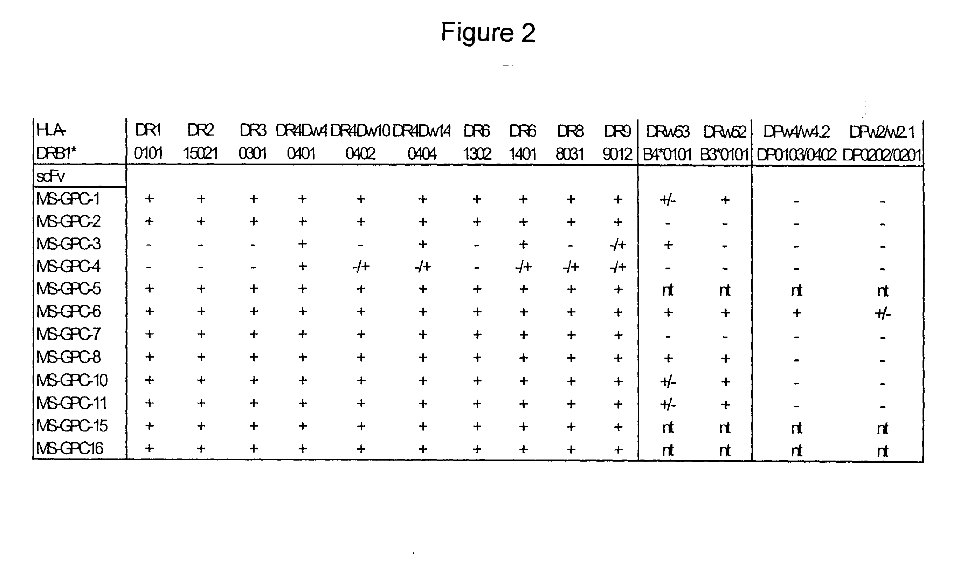

Figure 2 shows that virtually all anti-HLA DR antibody fragments react with all DRB1 allotypes tested. This observation taken together with the observation that all anti-HLA DR antibody fragments react with chimeric DR-IE, suggests that all anti-HLA DR antibody fragments (MS-GPC1 to 8, MS-GPC10 and 11, MS-GPC14 to 16) recognise the first domain of the monomorphic DRα chain or a monomorphic epitope on the first domain of the DRβ chain. Fragments MS-GPC-3 and 4 show less general reactivity but still react to a number of DRB1 allotypes.

Second, to further localise the epitope recognised by each anti-HLA DR antibody fragments, the fragment MS-GPC-8 was used as an example. MS-GPC-8 recognises all DR molecules, the DQ molecule but not the two DP molecules tested (Figure 2). A sequence comparison of α chains of these molecules and the computer model of the three dimensional structure of DR molecules revealed that the epitope localises in the C-terminal end of the α-helical region localised from Glu⁵⁵ to Tyr⁷⁹ of the N-terminal domain of the α-chain of HLA-DR.

Third, the PepSpot technique (US 6040423; Heiskanen et al., 1999) is used to create overlapping synthetic peptides corresponding to the sequence of the DRα1 and DRβ1 domain. Cross-reactivity tests between the MS-GPC-8 anti-HLA DR antibody fragment and this set of overlapping set of peptides localises the epitope for this antibody fragment more precisely within the α-chain or β-chain of the HLA-DR molecule.

### E. Killing activity of mAb fragments

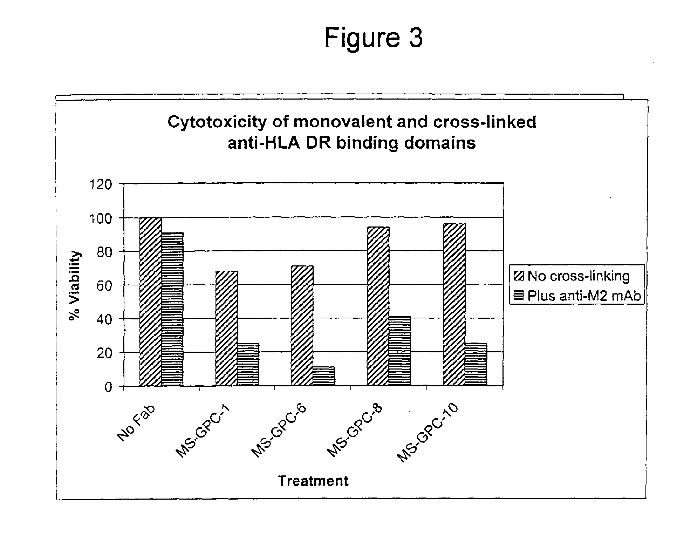

We demonstrated that a multivalent composition comprising of at least two anti-HLA DR antibody fragments caused killing of activated cells expressing the HLA-DR antigen. The killing efficiency of anti-HLA DR antibody fragments selected from the HuCAL library was tested on the HLA-DR positive tumour cell line GRANTA-519 (DSMZ, Germany). 2x10⁵ cells were incubated for 4 h at 37°C under 6% CO₂ with 200nM anti-HLA DR antibody fragments in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. Each anti-HLA DR antibody fragment was tested for its ability to kill activated cells as a monovalent anti-HLA DR antibody fragment or as a bivalent composition by the addition to 100 nM of a bivalent cross-linking anti-FLAG M2 mAb (Sigma). After 4 h incubation at 37°C under 6% CO₂, cell viability was determined by trypan blue staining and subsequent counting of remaining viable cells (Current Protocols in Immunology, 1997).
Anti-HLA DR antibody fragments from the HuCAL library showed much higher cytotoxic activity when cross-linked to form a bivalent composition by co-incubation with anti-FLAG M2 mAb (Figure 3). Incubation of cell lines alone or only in the presence of anti-FLAG M2 mAb without co-incubation of anti-HLA DR antibody fragments did not lead to cytotoxicity as measured by cell viability. We observe that higher order valences of the anti-HLA DR antibody fragments further decrease cell viability significantly. On addition to the incubation mix of Protein G, the multivalent complexes thus formed comprising anti-HLA DR antibody fragments, further decrease cell viability compared to the bivalent composition formed from incubation of the anti-HLA DR antibody fragments with only anti-FLAG M2 mAb.

Similar experiments show that other activated cell lines that express HLA-DR molecules were also killed using a cross-linked bivalent composition of the anti-HLA DR antibody Fab fragment MS-GPC8. Activated cell lines that show greater than 50% cell killing after 4h incubation include PRIESS, MHH-CALL4, MN 60, BJAB and BONNA-12.

Use of the Fab form of the anti-HLA DR antibody fragments MS-GPC-1, 6, 8 and 10 also shows similar cytotoxic activity to the above activated cells lines when formed as a bivalent composition using the cross-linking anti-FLAG M2 mAb.

The method described above to assay killing activity was used to determine the maximum killing capacity for each of the cross-liked bivalent anti-MHC DR antibody fragments against Priess cells. The maximum killing capacity observed for MS-GPC-1, MS-GPC-6, MS-GPC-8 & MS-GPC-10 was measured as 83%, 88%, 84% and 88% respectively.

### F. Killing selectivity of antigen-binding domains against a human antigen for activated versus non-activated cells

Human peripheral B cells are used to demonstrate that human anti-HLA-DR mAb-mediated cell killing is dependent on cell-activation. Around 50 ml of heparinised venous blood is taken from an HLA-DR typed healthy donor and fresh peripheral blood mononuclear cells (PBMC) are isolated by Ficoll-Hypaque Gradient Centrifugation (Histopaque-1077; Sigma) as described in Current Protocols in Immunology (John Wiley & Sons, Inc.; 1999). Purified B cells (∼5% of peripheral blood leukocytes) are obtained from around 5x10⁷ PBMCs using the B cell isolation kit and MACS LS⁺/VS⁺ columns (Miltenyi Biotec, Germany) according to manufacturers guidelines. Successful depletion of non-B cells is verified by FACS analysis of an aliquot of isolated B cells (HLA-DR positive and CD45 positive). Double staining and analysis is done with commercially available antibodies (Beckton Dickinson) using standard procedures as for example described in Current Protocols in Immunology (John Wiley & Sons, Inc.; 1999). An aliquot of the isolated B cells is tested for the ability of the cells to be activated by stimulation with Pokeweed mitogen (PWM) (Sigma) at a concentration of 2,5µg/ml in RPMI 1640 (PAA, Germany) supplemented with 5% human AB serum (Sigma, Germany), 2mM L-glutamine, 1% non-essential amino acids, 1mM sodium pyruvate and 0,1mg/ml kanamycin by incubation at 37°C under 6% CO₂ for four days. Successful activation is verified by standard procedures, for example morphology, by measuring ³H-thymidine uptake into the growing cells, or by FACS analysis of HLA-DR expression on the cell surface (Current Protocols in Immunology, John Wiley & Sons, Inc.; 1999).

The selectivity for killing of activated cells versus non-activated cells is demonstrated by incubating 1x10⁶/ml B cells activated as above compared to non-activated cells, respectively with 200nM of one the anti-HLA DR antibody fragments MS-GPC-1, 6, 8 & 10 together with 100nM of the cross-linking anti-FLAG M2 mAb in the medium described above but supplemented with 2,5% heat inactivated FBS (Biowhittaker Europe, Belgium) instead of human serum. After incubation at 37°C under 6% CO₂ for 1, 2, 4, and 6 h, cell viability is determined by fluorescein diacetate staining (FDA, Sigma) and subsequent counting of the green fluorescent cells using a fluorescence microscope (Leica, Germany) using standard procedures (Current Protocols in Immunology, 1997).

B cell activation is shown to be necessary for cell killing. After only 4 h following incubation with the cross-linked anti-HLA DR antibody fragments, over 50% of PWM activated B cells are killed. In contrast, non-activated B cells (not PWM stimulated) are not as susceptible to killing by the cross-linked anti-HLA DR antibody fragments - less than 15% dead cells can be seen after 4h.

We were surprised to see that our cross-linked anti-HLA-DR antibody fragments do not readily kill a particular tumour cell line. We hypothesised that although established as a stable cell line, cells in this culture were not sufficiently activated. Therefore, we conduct an experiment to stimulate activity of the MHH PREB1 cell line, using increased cell-surface expression of HLA-DR molecule as a marker of activation as follows.

Non-adherently growing MHH PREB1 cells are cultivated in RPMI medium containing the following additives (all from Gibco BRL and PAA): 10 % fetal calf serum (FCS), 2mM L-glutamine, 1% non-essential amino acids, 1 mM sodium pyruvate and 1x Gentamycin. Cells are activated to increase expression of HLA-DR molecule by incubation for two days with Lipopolysaccharide (LPS, Sigma, 10 µg/ml) and Interferon-gamma (IFN-γ, Roche, 40 ng/ml). The cell surface expression of HLA-DR molecules is monitored by flow cytometry with the FITC-conjugated mAb L243 (Becton Dickinson). Following activation, cell killing is performed for 4 h in the above medium but containing a reduced FCS concentration (2.5%) with a final antibody concentration of 200 nM.

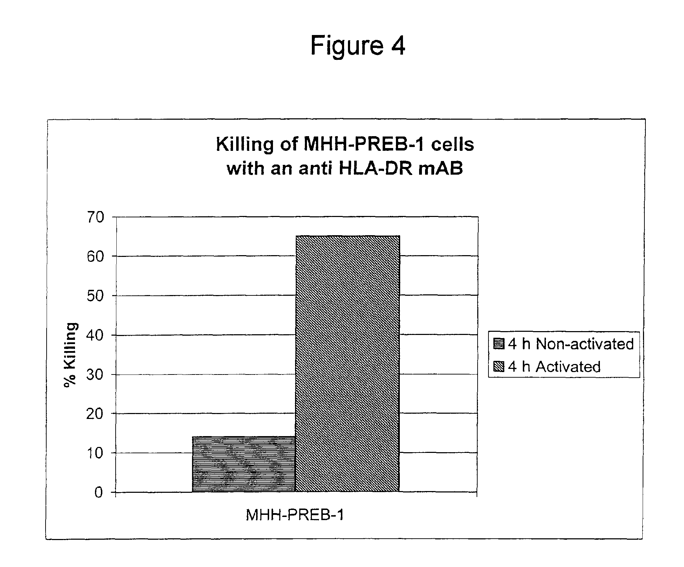

Incubation of MHH PREB1 for two days in the presence of LPS and IFN- γ results in a 2-fold increase in HLA-DR surface density (mean fluorescence shift from 123 to 260). Accordingly, the percentage of dead cells after 4 h incubation with 100 nM concentration of the IgG form of MS-GPC-8 (produced as described below) is greater than 60% compared to a non-activated culture for which less than 15% dead cells are observed (Figure 4). Viable cells are identified microscopically by exclusion of Trypan blue. Therefore, activation of MHH PREB1 cells by LPS and IFN-γ enhances the cell killing by binding of the IgG form of MS-GPC-8 to HLA DR molecules.

### G. Mechanism of cell-killing

The examples described above show that cell death occurs - needing only certain multivalent anti-HLA DR antibody fragments to cause killing of activated cells. No further cytotoxic entities or immunological mechanisms were needed to cause cell death, therefore demonstrating that cell death is mediated through an innate pre-programmed mechanism of the activated cell. The mechanism of apoptosis is a widely understood process of pre-programmed cell death. We were surprised by certain characteristics of the cell killing we observed that suggested the mechanism of killing for activated cells when exposed to our human anti-HLA DR antibody fragments was not apoptosis. For example, the speed at which we observed cells were killed appeared to be significantly faster than that reported for apoptosis. Two experiments are conducted to demonstrate that the mechanism of cell killing proceeds by a non-apoptotic mechanism.

First, we use Annexin-V-FITC and propidium iodide (PI) staining techniques to distinguish between apoptotic and non-apoptotic cell death - apoptotic cells (Annexin-V positive/PI negative) can be distinguished from dead (Annexin-V positive/PI positive) and fully functional cells (Annexin-V negative/PI negative) (using the manufactures recommended procedure). 1x10⁶/ml Priess cells are incubated at 37°C under 6%CO₂ with or without 200nM anti-HLA DR antibody fragment MS-GPC-8 together with 100 nM of the cross-linking anti-FLAG M2 mAb in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. To provide an apoptotic cell culture, 1x10⁶/ml Priess cells were induced to enter apoptosis by incubation in the above medium at 37°C under 6%CO₂ with 50µg/ml of the apoptosis-inducing anti-CD95 mAb DX2 (Pharmingen, California) cross-linked with 10µg/ml Protein-G (Sigma, Germany). At various incubation times (1, 15 and 60 min, 3 and 5 h) 200 µl samples are taken, washed twice and stained with Annexin-V-FITC (Pharmingen, California) and PI (Sigma, Germany) using Annexin-V binding buffer following the manufacturer's protocol. The amount of staining with Annexin-V-FITC and PI for each group of cells is analysed with a FACS Calibur (Beckton Dickinson, Germany).

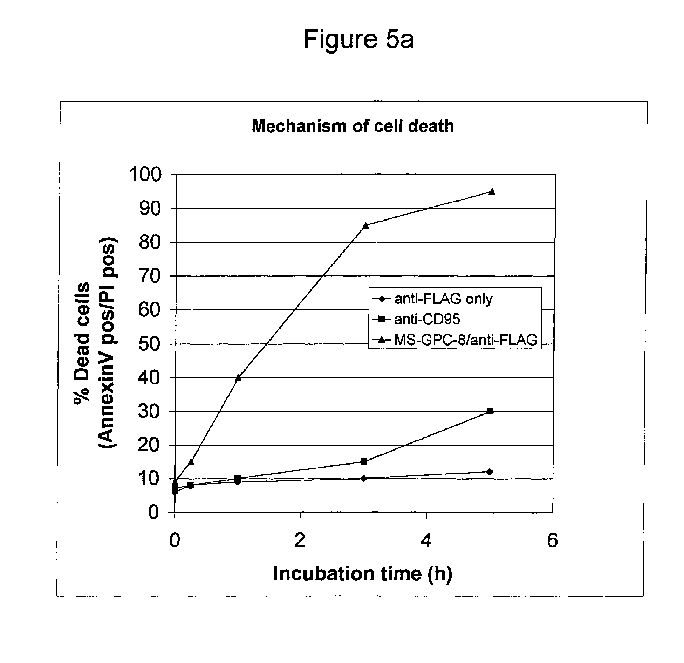

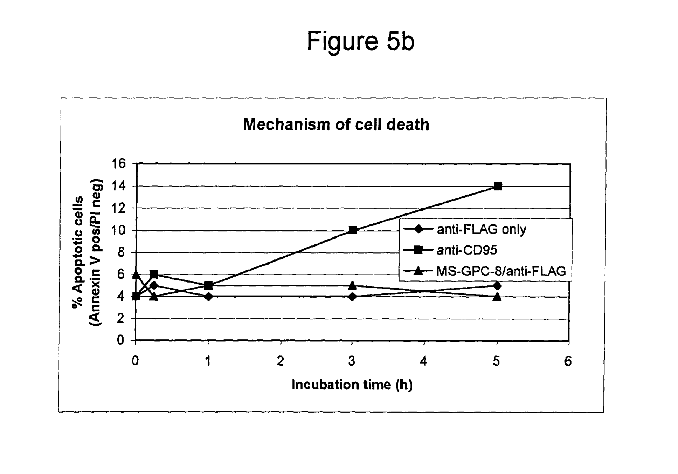

Cell death induced through the cross-linked anti-HLA DR antibody fragments shows a significantly different pattern of cell death than that of the anti-CD95 apoptosis inducing antibody or the cell culture incubated with anti-FLAG M2 mAb alone. The percentage of dead cells (as measured by Annexin-V positive/PI positive staining) for the anti-HLA DR antibody fragment/anti-FLAG M2 mAb treated cells increases far more rapidly than that of the anti-CD95 or the control cells (Figure 5a). In contrast, the percentage of apoptotic cells (as measured by Annexin-V positive/PI negative staining) increases more rapidly for the anti-CD95 treated cells compared to the cross-linked anti-HLA DR antibody fragments or the control cells (Figure 5b).

Second, we inhibit Caspase activity using zDEVD-fmk, an irreversible Caspase-3 inhibitor, and zVAD-fmk a broad spectrum Caspase inhibitor (both obtained from Bio-Rad). The mechanism of apoptosis is characterised by Caspase activity, and we hypothesised that if Caspases were not necessary for anti HLA-DR mediated cell death, we would observe no change in the viability of cells undergoing cell death in the presence of these Caspase inhibitors compared to those without. 2x10⁵ Priess cells are preincubated for 3 h at 37°C under 6% CO₂ with serial dilutions of the two caspase inhibitors ranging from 180 µM to 10 mM in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. HLA-DR mediated cell death is induced by adding 200nM of the human anti-HLA DR antibody fragment MS-GPC-8 and 100nM of the cross-linking anti-M2 mAb. An anti-CD95 induced apoptotic cell culture serves as a control for the activity of inhibitors (Drenou et al., 1999). After further incubation at 37°C and 6% CO₂, cell viability after 4 and 24 h is determined by trypan blue staining and subsequent counting of non-stained cells. As we hypothesised, cell viability of the anti-HLA DR treated cell culture is not significantly modified by the presence of the Caspase inhibitors, while cell death induced through anti-CD95 treatment is significantly decreased for the cell culture pre-incubated with the Caspase inhibitors. This observation supports our hypothesis that HLA-DR mediated cell death proceeds through a non-apoptotic mechanism that is independent of Caspase proteases.

### H. Affinity maturation

The Fab fragment MS-GPC-8-Fab (see C.1) is cloned via Xba/EcoRl from PMx9_Fab_GPC8 into pMORPH18_Fab, a phagemid-based vector for phage display of Fab fragments, to generate pMORPH18_Gab_GPC8 (see Fig. 9). The lambda chain pool is amplified from HuCAL-scFv in pMORPH13_scFv (see B.2 above and Figure 6) with PCR-primers CRT5 and CRT6 (which introduces a unique DraIII restriction endonuclease site) and cloned into pMORPH18_Fab_GPC8 cut with Nsil and DraIII (see vector map of pMORPH18_Fab_GPC8 in Fig. 9).

The Fab affinity maturation library is screened by two rounds of panning against MHC-II DRA*0101/DRB1*0401 as described in B.3 with the exception that in the second round the antigen concentration for coating is decreased to 12 ng/well. Optimised clones are identified by FACS as described above in B.5.

Table 2 contains the sequence characteristics of clones MS-GPC-8-6 and MS-GPC-8-17 that were identified using the affinity maturation procedure. The VH and VL families and the CDR3s listed refer to the HuCAL consensus-based antibody genes as described (Knappik et al., 2000). The full sequences of the VH and VL domains are shown in Figure 10.

The affinities of the identified optimised anti MHC class II antibody fragments are measured using the IgG form of these fragments. First each antibody fragment is converted to its IgG form as described below. Second, the affinities of the antigen binding domain when in IgG form is measured using standard BIAcore techniques (Biacore AG, Sweden). All measurements are conducted in HBS buffer (20 mM HEPES, 150 mM NaCI, pH 7.4) at a flow rate of 20 µl/mn at 25°c on a BIAcore 3000 instrument. Human MHC class II antigens DRA*0101/DRB*0401 are isolated according to example A and are diluted to 50-100 mg/ml in 100 mM sodium acetate. 500 and 4000 RU (reactive units) of the respective MHC class II molecules are immobilised on a BIAcore CM5 chip using standard EDC-NHS coupling chemistry with subsequent ethanolamine treatment. 5 different concentrations of the different IgG forms of the antigen binding domain are injected into the Biacore 3000 instrument and kₒₙ and k_{off} values are measured. Regeneration of the surface is achieved by using 10 mM glycine, pH 2.3 and 7.5 mM NaOH. Kₒₙ and k_{off} values are used to calculate the k_{D}-value. Improved affinity over non-optimised antigen binding domains is seen. Furthermore, these two optimised fragments showed other improved biological characteristics including specificity, reactivity and cytotoxic behaviours (data not shown).

### I. Generation of IgG

### I.1. Construction of HuCAL-immunoglobulin expression vectors

### a) Heavy chain cloning

The multiple cloning site of pcDNA3.1+ (Invitrogen) was removed (Nhel / Apal), and a stuffer compatible with the restriction sites used for HuCAL-design was inserted for the ligation of the leader sequences (Nhel / EcoRI), VH-domains (EcoRI / Blpl) and the immunoglobulin constant regions (Blpl /Apal). The leader sequence (EMBL M83133) was equipped with a Kozak sequence (Kozak, 1987). The constant regions of human IgG₁ (PIR J00228), IgG₄ (EMBL K01316) and serum IgA₁ (EMBL J00220) were dissected into overlapping oligonucleotides with lengths of about 70 bases. Silent mutations were introduced to remove restriction sites non-compatible with the HuCAL-design. The oligonucleotides were spliced by overlap extension-PCR.

### b) Light chain cloning

The multiple cloning site of pcDNA3.1/Zeo+ (Invitrogen) was replaced by two different stuffers. The κ-stuffer provided restriction sites for insertion of a κ-leader (Nhel / EcoRV), HuCAL-scFv Vκ-domains (EcoRV / BsiWI) and the κ-chain constant region (BsiWI / Apal). The corresponding restriction sites in the λ-stuffer were Nhel / EcoRV (λ-leader), EcoRV / Hpal (Vλ- domains) and Hpal / Apal (λ-chain constant region). The κ-leader (EMBL Z00022) as well as the λ-leader (EMBL L27692) were both equipped with Kozak sequences. The constant regions of the human κ- (EMBL J00241) and λ -chain (EMBL M18645) were assembled by overlap extension-PCR as described above.

### 1.2. Generation of IgG-expressing CHO-cells

All cells were maintained at 37°C in a humidified atmosphere with 5% CO₂ in media recommended by the supplier. CHO-K1 (CRL-9618) were from ATCC and were co-transfected with an equimolar mixture of IgG heavy and light chain expression vectors. Double-resistant transfectants were selected with 600 µg/ml G418 and 300 µg/ml Zeocin (Invitrogen) followed by limiting dilution. The supernatant of single clones was assessed for IgG expression by capture-ELISA (see below). Positive clones were expanded in RPMI-1640 medium supplemented with 10% ultra-low IgG-FCS (Life Technologies). After adjusting the pH of the supematant to 8.0 and sterile filtration, the solution was subjected to standard protein A column chromatography (Poros 20A, PE Biosystems).

The IgG forms of anti-HLA DR antigen binding domains show improved characteristics over the antibody fragments. For example, the IC50 value of the IgG form of MS-GPC-8 was 10 nM and the maximum killing capacity determined as 84% for MHH-Call4 and 89% for GRANTA 519. Note that all IC50 concentrations given (also for Fab and scFv fragments) are shown as the molar equivalent of the bivalent IgG to simplify comparison.

### J. Immunosuppression using an HLA-DR specific antibody measured by T cell proliferation

The IgG and monovalent forms of the anti-HLA DR antibody fragment MS-GPC-8 prepared as described above can be assayed for their ability to inhibit the proliferative T cell response of antigen-primed lymph node cells from mice carrying a chimeric mouse-human class II transgene with an RA-associated peptide binding site, and lack murine class II molecules (Muller et al., 1990; Woods et al., 1994; Current Protocols in Immunology, Vol. 2, 7.21; Ito et al., 1996). Here, the immunisation takes place *in vivo,* but the inhibition and readout are *ex vivo.* Transgenic mice expressing MHC-II molecules with binding sites of two RA associate molecules, DRB1*0401 and 0404 were previously generated (Ito et al., 1996). The mice lack murine MHC-class II, and thus, all Th responses are channelled through a single human RA-associated MHC-class II molecule (Ito et al. 1996). These transgenic mice represent a model for testing human class II antagonist.

The inhibitory effect of the anti-HLA DR antibody fragment MS-GPC-8 and its IgG form on T-cell proliferation is measured using chimeric T-cells and antigen presenting cells isolated from the lymph nodes of chimeric 0401-IE and 0404-IE transgenic mice previously immunised with hen egg lysozyme and ovalbumin (Ito et al. 1996), respectively. 1.5x10⁵ cells are incubated in 0.2 ml wells of 96-well tissue culture plates in the presence of antigen (half-maximal stimulatory concentration) and either the anti-HLA DR antibody fragment MS-GPC-8 or its IgG form (1 nM - 200 nM) in serum free HL-1 medium containing 2 mM L-glutamine and 0.1 g/l Kanamycin for three days. Antigen specific proliferation is measured by ³H-methyl-thymidin incorporation during the last 16h of culture (Falcioni et al., 1999). Inhibition of T-cell proliferation on treatment with the anti-HLA DR antibody fragment and its IgG form may be observed by comparison to control wells containing antigen.

### K. Selection of useful peptide/protein for treatment of disease of the immune system

In order to select the most appropriate protein/peptide to enter further experiments to assess its suitability for use in a therapeutic composition for the treatment of diseases of the immune system, additional data is collected. Such data for each monovalent antibody fragment or IgG form of the anti-HLA antigen antibody fragments can include the affinity, reactivity, specificity, EC50-values (effective concentration to achieve 50% effectiveness) and inhibition of T-cell proliferation data, or *in vitro* killing efficiency as measured by IC50 and the maximal percentage cell killing as estimated *in vitro.*

The antibody fragment or IgG form of the anti-HLA antigen antibody fragments that shows the lowest EC50, highest affinity, highest killing, best specificity and/or greatest inhibition of T-cell inhibition might be chosen to enter further experiments. Such experiments may include, for example, phase I clinical trials in humans.

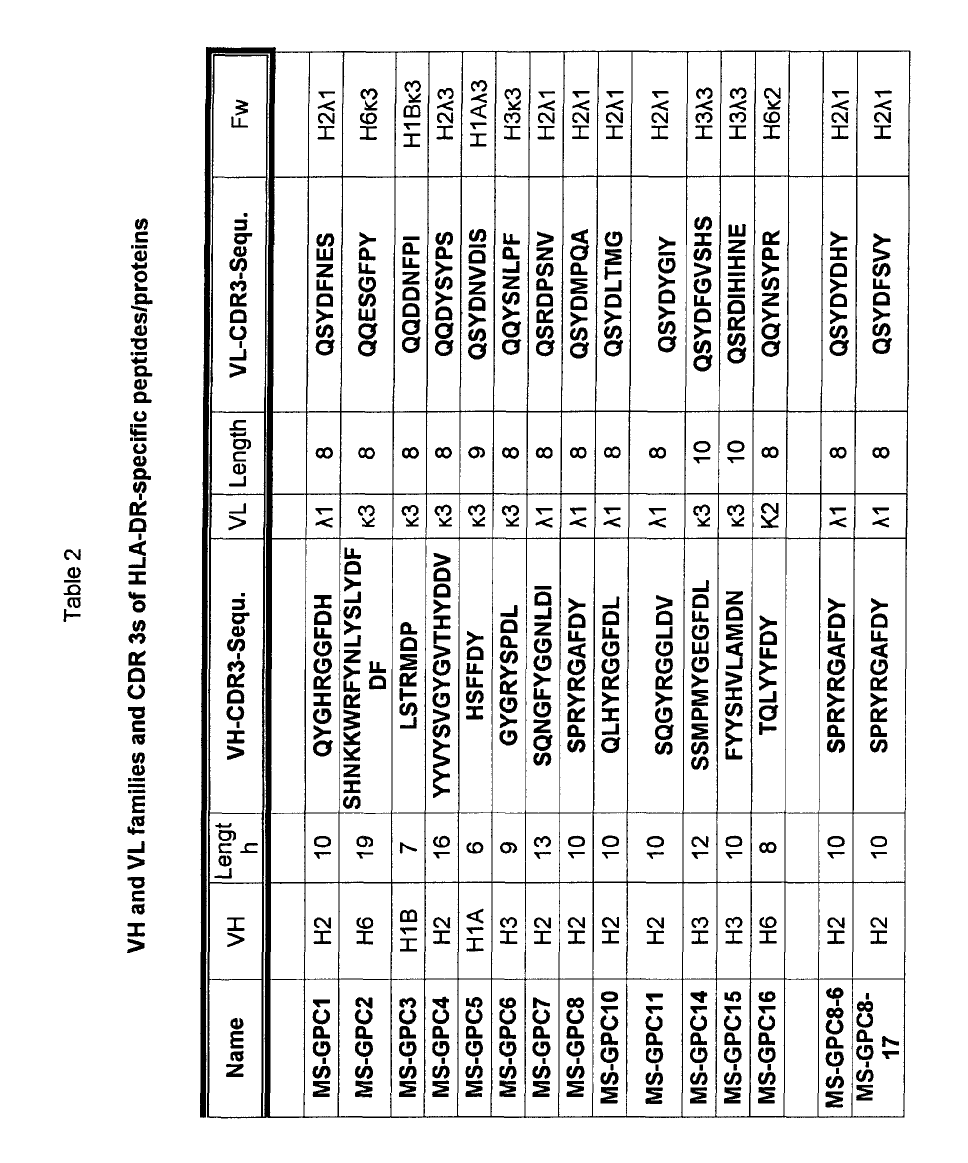

### References

Ausubel, F.M., Brent, R., Kingston, R.E., Moore, D.D., Seidman, J.G., Smith, J.A. and Struhl, K. (1998) Current protocols in molecular biology. John Wiley & Sons, Inc., New York, U.S.A.

Babbitt B, Allen PM, Matsueda G, Habe E, Unanue ER, (1985), Nature 317:359.

Baxevanis, C.N., Wernet, D., Nagy, Z.A., Maurer, P.H., and Klein, J. (1980). Immunogenetics, 11, 617.

Billing, R., and Chatterjee, S. (1983). Transplant. Proc. 15, 649.

Bird, R.E. et al. Single-chain antigen-binding proteins [published erratum appears in Science 1989 Apr 28;244(4903):409]. Science 242, 423-6 (1988).

Bonagura, V.R., Ma, a., McDowell, J., Lewison, A., King, D.W. and Suciu-Foca, N. (1987). Cell. Immunolo., 108(2), 356.

Brinkmann, U., Reiter, Y., Jung, S., Lee, B. & Pastan, I. (1993). A recombinant immunotoxin containing a disulfide-stabilized Fv fragment. Proc. Natl. Acad. Sci. U.S.A. 90, 7538-7542.

Brown JH, Jardetsky TS, Gorga JC, Stern LJ, Urban RG, Strominger JL, Wiley DC., (1993), Nature 364: 33.

Current Protocols in Immunology, Vol. 2, 7.21.

Drenou B, Blancheteau V, Burgess DH, Fauchet R, Charron DJ, Mooney NA., (1999), J. Immunol. 163: 4115.

Falcioni et al. (1999). Nat Biotechnol. 17: 562-567.

Glockshuber, R., Malia, M., Pfitzinger, I. & Plückthun, A. (1990). A comparison of strategies to stabilize immunoglobulin Fv-fragments. Biochemistry 29, 1362-1367.

Gorga J.C., Foran, J., Burakoff, S.J., Strominger, J.L., (1984) Meth Emzym., 108, 607-613.

Gorga, J.C., Horejsi, V., Johnson, D.R., Raghupathy, R., Strominger, J.L., J.Biol. Chem. 262 (1987)16087-94.

Gorga, J.C., Knudsen, P.J., Foran, J.A., Strominger, J.L., Burakoff, S.J., (1986), Cell. Immunol. 103 160-73.

Heiskanen T, Lundkvist A, Soliymani R, Koivunen E, Vaheri A, Lankinen H (1999) Virology, 262(2), 321.

Hopp, T.P., Prickett, K.S., Price, V.L., Libby, R.T., March, C.J., Cerretti, D.P., Urdal, D.L. & Conlon, P.J. (1988), Bio/Technology 6,1204-1210.

Huston, J.S. et al. Protein engineering of antibody binding sites: recovery of specific activity in an anti-digoxin single-chain Fv analogue produced in Escherichia coli. Proc Natl Acad Sci U S A 85, 5879-83 (1988).

Ito K, Bian H.-J, Molina M, Han J, Magram J, Saar E, Belunis C, Bolin DR, Arceo R, Campbell R, Falcioni F, Vidovic' D, Nagy ZA., (1996), J. Exp. Med. 183: 2635-2644.

Jones et al., (1986), Nature 321: 522-525.

Jonker, M., Schellekens, P.T., Harpprecht, J., and Slingerland, W. (1991), Transplant. Proc., 23, 264.

Jonker, M., van Lambalgen, R., Mitchell, D.J., Durham, S.K., and Steinman, L. (1988), Autoimmunity, 1, 399.

Kahoury E.L. and Marshall L.A., (1990) Cell. Tissue Res., 262(2):217-24

Klohe EP, Watts R, Bahl M, Alber C, Yu W-Y, Anderson R, Silver J, Gregersen PK, Karr RK., (1988), J. Immunol. 141: 2158-2164.

Knappik, A. & Plückthun, A., (1994), Biotechniques 17, 754-761.

Knappik, A., Ge, L., Honegger, A., Pack, P., Fischer, M., Wellnhofer, G., Hoess A., Wölle, J., Plückthun, A. and Virnekäs, B., (2000), J. Mol. Biol. 296, 55.

Kozak, M. (1987) J. Mol. Biol. 196, 947.

Kuby, J. Immunology:1994, 2^{nd} edition.

McMichael, S.J., Sasazuki, T., McDevitt, H.O., and Payne, R.O., (1977), Arthritis Rheum., 20, 1037.

Muller et al., (1990), J. Immunol., 145: 4006.

Naquet, P., Marchetto, S., and Pierres, M., (1983), Immunogenetics, 18, 559.

Nepom, G.T., Benacerraf, B., and Germain, R.N., (1981), J. Immunol., 127, 31.

Nepom, G.T., Byers, P., Seyfried, C., Healey, L.A., Wilske, K.R., Stage, D., and Nepom, B.S., (1989), Arthritis Rheum., 32,15.

Ohta, N., Nishimura, Y.K., Tanimoto, K., Horiuchi, Y., Abe, C., Shiokawa, Y., Abe, T., Katigari, M., Yoshiki, T., and Sasazuki, T., (1982), Hum. Immunol., 5, 123.

Otten et al (1997) pp 5.4.1 - 5.4.19 in Current Protocols in Immunology, Eds. Coligan et al. Green & Wiley, New York.

Pack, P. and Plückthun, A., (1992), Biochemistry 31, 1579-1584.

Pack, P., (1994), Ph.D. thesis, Ludwig-Maximilians-Universität München.

Pack, P., Kujau, M., Schroeckh, V., Knüpfer, U., Wenderoth, R., Riesenberg D. and Plückthun, A. (1993), Bio/Technology 11, 1271-1277.

Presta, (1992), Curr. Op. Struct. Biol. 2: 593-596.

Riechmann et al., (1988), Nature 332: 323-329.

Rheinnecker, M., Hardt, C., Ilag, L.L., Kufer, P., Gruber, R., Hoess, A., Lupas, A., Rottenberger, C., Plückthun, A. and Pack, P., (1996), J. Immunol. 157, 2989.

Rosenbaum, J.T., Adelman, N.E., and McDevitt, H.O., (1983), J. Exp. Med., 154, 1694.

Sambrook et al., 1989, Molecular Cloning: a Laboratory Manual, 2nd ed.

Schiff, B., Mizrachi, Y., Orgad, S., Yaron, M., and Gazit, I., (1982), Ann. Rheum. Dis., 41, 403.

Schmidt, T. G. M. & Skerra, A. (1993). Prot. Engineering 6, 109-122.

Schmidt, T. G. M. & Skerra, A. (1994). J. Chromatogr. A 676, 337-345.

Schmidt, T. G. M. et al. (1996). J. Mol. Biol. 255, 753-766.

Skerra, A. and Plückthun, A. (1988). Science 240, 1038.

Smith, R.M., Morgan, A., and Wraith, D.C. (1994). Immunology, 83, 1.

Stasny, P. (1978). N. Engl. J. Med., 298, 869.

Stausbøl-Gnøn, B., Wind, T., Kjær, S., Kahns, L., Hansen, N.J.V., Kristensen, P. and Clark, B.F.C. (1996) FEBS Lett. 391, 71.

Stem, A.S: and Podlaski, F.J, (1993) Techniques in Protein Chemistry IV, Academic Press Inc., San Diego, CA.

Stevens, H.P., Roche, N., Hovius, S.E., and Jonker, M., (1990), Transplant. Proc., 22, 1783.

Tiwari, J., and Terasaki, P., (1985), HLA and disease association. (New York: springer Verlag).

Vidovic D, Falcioni F, Bolin DR, Nagy ZA., (1995a), Eur. J. Immunol., 25: 1326.

Vidovic D, Falcioni F, Siklodi B, Belunis CJ, Bolin DR, Ito K, Nagy ZA., (1995b), Eur J. Immunol., 25:3349.

Voss, S. & Skerra, A. (1997). Protein Eng. 10, 975-982.

Waldor, M.K., Sriram, S., McDevitt, H.O., and Steinman, L. (1983). Proc. Natl. Acad. Sci. USA, 80, 2713.

Winter, G., Griffiths, A.D., Hawkins, R.E. and Hoogenboom, H.R. (1994) Making antibodies by phage display technology. Annu. Rev. Immunol. 12, 433.

Woods et al., (1994), J Exp Med. 180: 173-81.

## Claims

1. A peptide/protein comprising at least one antibody-based antigen-binding domain of human composition with a binding specificity for a human MHC class II antigen, wherein binding of said peptide/protein to said antigen expressed on the surface of a cell causes or leads to modulation of the immune response.

2. The peptide/protein of claim 1, wherein said cells are lymphoid cells.

3. The peptide/protein of claim 1, wherein said cells are non-lymphoid cells and express MHC class II molecules.

4. The peptide/protein of any one of claims 1 to 3, wherein said human MHC class II antigen comprises epitopes selected from HLA-DR molecules.

5. The peptide/protein of claim 4, wherein said antigen-binding domain binds to at least one epitope of the α-chain of HLA-DR.

6. The peptide/protein of claim 5, wherein said antigen-binding domain binds to at least one epitope of the first domain of the α-chain of HLA-DR.

7. The peptide/protein of claim 6, wherein said antigen-binding domain binds to at least one epitope on the α-helix ranging from Glu55 to Tyr79 of the α-chain of HLA-DR.

8. The peptide/protein of claim 4, wherein said antigen-binding domain binds to at least one epitope of the β-chain of HLA-DR.

9. The peptide/protein of claim 8, wherein said antigen-binding domain binds to at least one epitope of the first domain of the β-chain of HLA-DR.

10. The peptide of any one of claims 1 to 9, wherein the IgG-form of said antigen-binding domain binds to said antigen with an affinity of less than 50 nM.

11. The peptide/protein of claim 10, wherein the IgG-form of said antigen-binding domain binds to said antigen with an affinity of less than 10 nM.

12. The peptide/protein of any one of claims 1 to 11, wherein said antigen-binding domain comprises a monavalent antibody fragment selected from Fv, scFv, dsFv and Fab fragment.

13. The peptide/protein of any one of claims 1 to 12, comprising an (Fab')₂ antibody fragment or a mini-antibody fragment.

14. The peptide/protein of any one of claims 1 to 11, comprising a full antibody selected from IgG1, IgG2a, IgG2b, IgG3, IgG4, IgA and IgM.

15. The peptide/protein of any one of claims 1 to 14, wherein said modulation of the immune response causes or leads to immunosuppression.

16. The peptide/protein of claim 15, wherein said immunosuppression is brought about by down-regulation of expression of said MHC class II antigen on said cells.

17. The peptide/protein of claim 15, wherein said immunosuppression is brought about by inhibition of the interaction between said cells, wherein said interaction would normally lead to or cause an immune response.

18. The peptide/protein of claim 15, wherein said immunosuppression is brought about by killing of said cells.

19. The peptide/protein of claim 18, wherein said killing is mediated by binding of a multivalent composition of one or more of said peptide/proteins to said antigen expressed by said cells.

20. The peptide/protein of claim 18 or 19, wherein said killing affects at least 50%, preferably at least 80%, of activated cells compared to killing of less than 15%, preferably less than 10%, of non-activated cells.

21. The peptide/protein of any one of claims 18 to 20, wherein said killing is an innate pre-programmed process of said cell.

22. The peptide/protein claim 21, wherein said killing is non-apoptotic.

23. The peptide/protein of claim 22, wherein said killing is dependent on the action of non-caspase proteases.

24. The peptide/protein of any one of claims 1 to 23, wherein said antigen-binding domain consists of a combination of a VH domain and a VL domain, wherein said combination is selected from the clones MS-GPC1 to 8, MS-GPC10 to 11, MS-GPC14, MS-GPC15, MS-GPC16, MS-GPC8-6, and MS-GPC8-17.

25. The peptide/protein of any one of claims 1 to 24, wherein said antigen-binding domain consists of a combination of HuCAL VH2 and HuCAL Vλ1, wherein the VH CDR3 sequence is taken from the consensus CDR3 sequence
nnnnRGnFDn
wherein each n independently represents any amino acid residue; and
wherein the VL CDR3 sequence is taken from the consensus CDR3 sequence
QSYDnnnn
wherein each n independently represents any amino acid residue.

26. A nucleic acid encoding an antigen-binding peptide/protein of any one of claims 1 to 25.

27. A variant of the nucleic acid of claim 26 which hybridises under stringent conditions with a nucleic acid sequence of claim 26, wherein said variant encodes a peptide/protein according to any one of claims 1 to 25.

28. A vector comprising at least one nucleic acid of claim 26 and/or at least one variant thereof according to claim 27.

29. A host cell harbouring at least one nucleic acid of claim 26 and/or at least one variant thereof according to claim 27 and/or at least one vector of claim 28.

30. A method for the production of an antigen-binding peptide/protein of any one of claims 1 to 25 comprising the expression of at least one nucleic acid according to claim 26 and/or at least one variant thereof according to claim 27 in a suitable expression system, preferably in a host cell.

31. A pharmaceutical composition comprising at least one antigen-binding peptide/protein of any one of claims 1 to 25 and, optionally, a pharmaceutically acceptable carrier and/or diluent.

32. The use of an antigen-binding peptide/protein of any one of claims 1 to 25 for the preparation of a pharmaceutical composition for the treatment of animals.

33. The use according to claim 32, wherein said animal is a human.

34. The use according to claim 32 or 33, wherein said treatment is the treatment or prevention of a condition **characterised by** MHC class II-mediated activation of T and/or B cells.

35. The use according to claim 32 or 33, wherein said treatment is the treatment or prevention of a condition **characterised by** expression of MHC class II expression at pathological sites of inflammation.

36. The use according to any one of claims 32 to 35 wherein said treatment is the treatment or prevention of diseases of the immune system.

37. The use according to any one of claims 32 to 36, wherein said treatment is the treatment or prevention of a condition selected from rheumatoid arthritis, juvenile arthritis, multiple sclerosis, Grave's disease, narcolepsy, psoriasis, systemic lupus erythematosus, transplant rejection, graft vs. host disease, Hashimoto's disease, myasthenia gravis, pemphigus vulgaris, glomerulonephritis, thyroiditis, insulitis, primary biliary cirrhosis, irritable bowel disease and Sjogren syndrome.

38. The use according to claim 37, wherein said treatment is the treatment or prevention of a condition selected from systemic lupus erythematosus, myasthenia gravis, multiple sclerosis, transplant rejection, graft vs. host disease and rheumatoid arthritis.

39. A diagnostic composition containing at least one antigen-binding peptide/protein of any one of claims 1 to 25.

40. A method for immunosuppression comprising the step of contacting a lymphoid cell or a non-lymphoid cell which expresses MHC class II molecules with at least one peptide/protein according to any one of the claims 1 to 25.

41. A method for immunosuppression according to claim 40, wherein said immunosuppression is brought about by down-regulation of expression of said MHC class II antigen on said cells.

42. A method for immunosuppression according to claim 40, wherein said immunosuppression is brought about by inhibition of the interaction between said cells, wherein said interaction would normally lead to or cause an immune response.

43. A method for selective immunosuppression according to claim 40, wherein said immunosuppression is brought about by killing of said cells.

44. A diagnostic composition containing at least one nucleic acid of claim 26 and/or a variant thereof according to claim 27.

45. A kit comprising
(i) a peptide/protein according to any one of claims 1 to 25, and
(ii) a detectable moiety or moieties, and
(iii) reagents and/or solutions to effect and/or detect binding of (i) to an antigen.
